(19) **Europäisches Patentamt / European Patent Office / Office européen des brevets**

(11) **EP 1 444 357 B1**

(12) **EUROPÄISCHE PATENTSCHRIFT**

(45) Veröffentlichungstag und Bekanntmachung des Hinweises auf die Patenterteilung:
**12.01.2011 Patentblatt 2011/02**

(21) Anmeldenummer: **02803744.8**

(22) Anmeldetag: **11.11.2002**

(51) Int Cl.:
*C12Q 1/68* (2006.01)     *G01N 33/543* (2006.01)

(86) Internationale Anmeldenummer:
**PCT/DE2002/004171**

(87) Internationale Veröffentlichungsnummer:
**WO 2003/046209 (05.06.2003 Gazette 2003/23)**

(54) **VERFAHREN ZUM ERFASSEN VON NUKLEINSÄUREMOLEKÜLEN MITTELS MINDESTENS EINER EINHEIT ZUM IMMOBILISIEREN VON NUKLEINSÄUREMOLEKÜLEN UND BIOSENSOR ZUM ERFASSEN VON NUKLEINSÄUREMOLEKÜLEN**

METHOD FOR DETECTING NUCLEIC ACIDS USING AT LEAST ONE UNIT FOR IMMOBILIZING NUCLEIC ACIDS AND A BIOSENSOR FOR DETECTING NUCLEIC ACIDS

PROCEDE DE DETECTION D'ACIDES NUCLEIQUES A L'AIDE D'AU MOINS UNE UNITE POUR IMMOBILISER DES ACIDES NUCLEIQUES ET BIOCAPTEUR APPROPRIE POUR DETECTER DES ACIDES NUCLEIQUES

(84) Benannte Vertragsstaaten:
**DE FR GB**

(30) Priorität: **14.11.2001 DE 10155892**

(43) Veröffentlichungstag der Anmeldung:
**11.08.2004 Patentblatt 2004/33**

(73) Patentinhaber: **SIEMENS AKTIENGESELLSCHAFT**
**80333 München (DE)**

(72) Erfinder:
• **PAULUS, Christian**
**82362 Weilheim (DE)**
• **SCHINDLER-BAUER, Petra**
**81375 München (DE)**

(74) Vertreter: **Viering, Jentschura & Partner**
**Postfach 22 14 43**
**80504 München (DE)**

(56) Entgegenhaltungen:
WO-A-99/53093     DE-A- 19 926 457
US-A- 5 312 527     US-A- 5 972 692
US-A- 6 017 696     US-B1- 6 168 948

• WETMUR J G: "DNA PROBES: APPLICATIONS OF THE PRINCIPLES OF NUCLEI ACID HYBRIDIZATION" CRITICAL REVIEWS IN BIOCHEMISTRY AND MOLECULAR BIOLOGY, CRC PRESS, BOCA RATON, FL, US, vol. 26, no. 3/04, 1 January 1991 (1991-01-01), pages 227-259, XP000616490 ISSN: 1040-9238

**Beschreibung**

Die Erfindung betrifft Verfahren zum Erfassen von makromolekularen Biopolymeren mittels mindestens einer Einheit zum Immobilisieren von makromolekularen Biopolymeren sowie einen Biosensor zum Erfassen von makromolekularen Biopolymeren.

Aus [1] bis [4] sind Verfahren zum Erfassen von DNA-Molekülen bekannt, bei denen zur Erfassung Biosensoren eingesetzt werden, die auf Elektrodenanordnungen basieren.

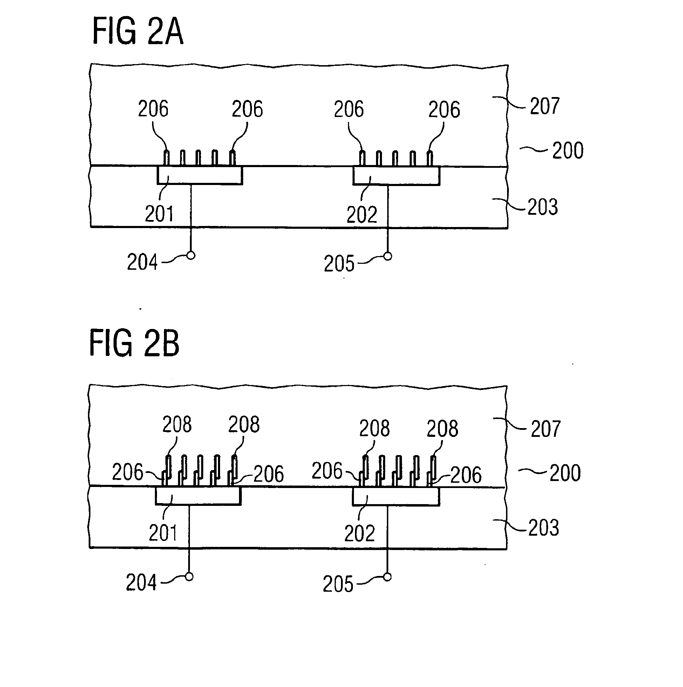

**Fig.2a** und **Fig.2b** zeigen einen solchen Sensor, wie er in [1] und [4] beschrieben ist. Der Sensor 200 weist zwei Elektroden 201, 202 aus Gold auf, die in einer Isolatorschicht 203 aus Isolatormaterial eingebettet sind. An die Elektroden 201, 202 sind Elektroden-Anschlüsse 204, 205 angeschlossen, an denen das an der Elektrode 201, 202 anliegende elektrische Potential zugeführt werden kann. Die Elektroden 201, 202 sind als Planarelektroden angeordnet. Auf jeder Elektrode 201, 202 sind DNA-Sondenmoleküle 206 immobilisiert (vgl. **Fig.2a**). Die Immobilisierung erfolgt gemäß der sogenannten Gold-Schwefel-Kopplung. Auf den Elektroden 201, 202 ist der zu untersuchende Analyt 207, aufgebracht. Der Analyt kann dabei beispielsweise eine elektrolytische Lösung verschiedener DNA-Sequenzen sein.

Sind in dem Analyt 207 DNA-Stränge 208 mit einer Sequenz enthalten, die zu der Sequenz der DNA-Sondenmoleküle 206 komplementär ist, so hybridisieren diese DNA-Stränge 208 mit den DNA-Sondenmolekülen 206 (vgl. **Fig. 2b**).

Eine Hybridisierung eines DNA-Sondenmoleküls 206 und eines DNA-Strangs 208 findet nur dann statt, wenn die Sequenzen des jeweiligen DNA-Sondenmoleküls 206 und des entsprechenden DNA-Strangs 208 zueinander komplementär sind. Ist dies nicht der Fall, so findet keine Hybridisierung statt. Somit ist ein DNA-Sondenmolekül einer vorgegebenen Sequenz jeweils nur in der Lage einen bestimmten, nämlich den DNA-Strang mit jeweils komplementärer Sequenz zu binden, d.h. mit ihm zu hybridisieren.

Findet eine Hybridisierung statt, so verändert sich, wie aus **Fig.2b** ersichtlich, neben anderen elektrischer Parameter auch die Kapazität zwischen den Elektroden. Diese Änderung der Kapazität kann als Messgröße für die Erfassung von DNA-Molekülen herangezogen werden.

Aus [5] ist eine weitere Vorgehensweise für die Untersuchung des Elektrolyts auf die Existenz eines DNA-Strangs mit vorgegebener Sequenz bekannt. Bei dieser Vorgehensweise werden die DNA-Stränge der gewünschten Sequenz mit einem Fluoreszenz-Farbstoff markiert und deren Existenz wird anhand der Fluoreszenzeigenschaften der markierten Moleküle bestimmt. Hierzu wird Licht beispielsweise im sichtbaren oder ultravioletten Wellenlängenbereich auf den Elektrolyten gestrahlt und es wird das von dem Analyten, insbesondere von dem nachzuweisenden markierten DNA-Strang, emittierte Fluoreszenzlicht erfasst. Aufgrund des Fluoreszenzverhaltens, d.h. insbesondere aufgrund der erfassten, emittierten Lichtstrahlen wird bestimmt, ob der nachzuweisende DNA-Strang mit der entsprechend vorgegebenen Sequenz in dem Analyten enthalten ist oder nicht.

Diese Vorgehensweise ist sehr aufwendig, da eine sehr genaue Kenntnis über das Fluoreszenzverhalten des entsprechenden Markermoleküls am DNA-Strang erforderlich ist und weiterhin eine Markierungsreaktion der DNA-Stränge vor Beginn des Verfahrens notwendig ist. Ferner ist eine sehr genaue Justierung des Erfassungsmittels zum Erfassen der emittierten Lichtstrahlen erforderlich, damit die Lichtstrahlen überhaupt erfasst werden können.

Somit ist diese Vorgehensweise teuer, kompliziert sowie gegen Störeinflüsse sehr empfindlich, wodurch das Messergebnis sehr leicht verfälscht werden kann.

Weiterhin ist ein Reduktions-/Oxidations-Recycling-Verfahren zum Erfassen makromolekularer Biopolymere aus [2] und [3] bekannt.

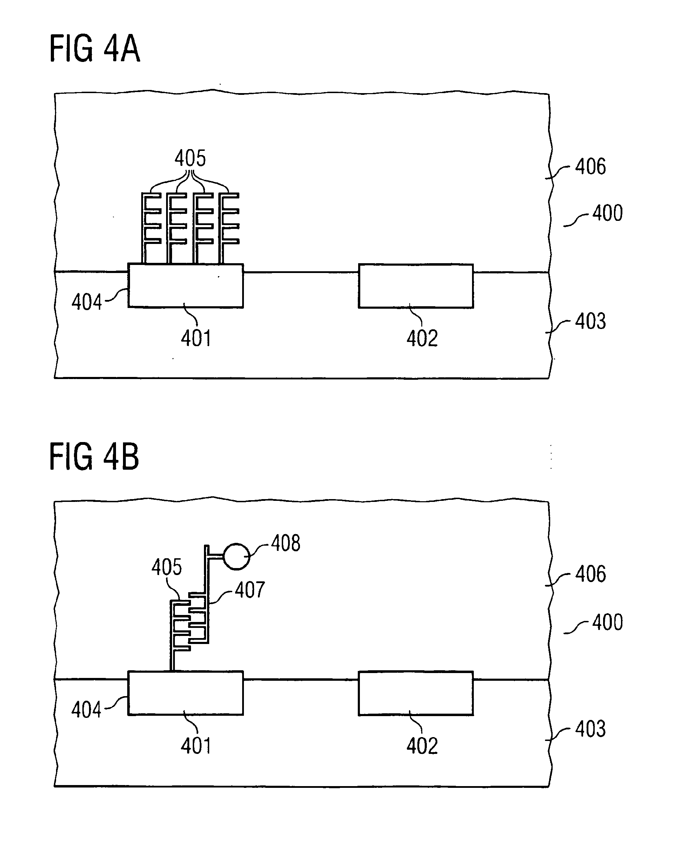

Das Reduktions-/Oxidations-Recycling-Verfahren, im weiteren auch als Redox-Recycling-Verfahren bezeichnet, wird im weiteren anhand der **Fig.4a** bis **Fig.4c** näher erläutert.

**Fig.4a** zeigt einen Biosensor 400 mit einer ersten Elektrode 401 und einer zweiten Elektrode 402, die auf einem Substrat 403 als Isolatorschicht aufgebracht sind.

Auf der ersten Elektrode 401 aus Gold ist ein Haltebereich, ausgestaltet als Halteschicht 404, aufgebracht. Der Haltebereich dient zum Immobilisieren von DNA-Sondenmolekülen 405 auf der ersten Elektrode 401.

Auf der zweiten Elektrode ist kein solcher Haltebereich vorgesehen.

Sollen mittels des Biosensors 400 DNA-Stränge mit einer Sequenz erfasst werden, die komplementär zu der Sequenz der DNA-Sondenmoleküle 405 ist, so wird der Sensor 400 mit einer zu untersuchenden Lösung 406 derart in Kontakt gebracht, dass in der zu untersuchenden Lösung 406 eventuell enthaltene DNA-Stränge mit der komplementären Sequenz zu der Sequenz der DNA-Sondenmoleküle 405 hybridisieren können.

**Fig.4b** zeigt den Fall, dass in der zu untersuchenden Lösung 406 die zu erfassenden DNA-Stränge 407 enthalten sind und an die DNA-Sondenmoleküle 405 hybridisiert sind.

Die DNA-Stränge 407 in der zu untersuchenden Lösung sind mit einem Enzym 408 markiert, mit dem es möglich ist, im weiteren beschriebene Moleküle in Teilmoleküle zu spalten.

Üblicherweise ist eine erheblich größere Anzahl von DNA-Sondenmolekülen 405 vorgesehen, als zu ermittelnde DNA-Stränge 407 in der zu untersuchenden Lösung 406 enthalten sind.

Nachdem die in der zu untersuchenden Lösung 406 eventuell enthaltenen DNA-Stränge 407 mit dem Enzym 408 mit den immobilisierten DNA-Sondenmolekülen hybridisiert haben, erfolgt eine Spülung des Biosensors 400, wodurch die nicht hybridisierten DNA-Stränge entfernt werden und der Biosensor 400 von der zu untersuchenden Lösung 406 gereinigt wird. Dieser zur Spülung verwendeten Spüllösung oder in einer weiteren Phase eigens zugeführten weiteren Lösung 412 wird eine elektrisch ungeladene Substanz beigegeben, die Moleküle enthält, die durch das Enzym an den hybridisierten DNA-Strängen 407 in ein erstes Teilmolekül einer negativen ersten elektrischen Ladung und in ein zweites Teilmolekül einer positiven zweiten elektrischen Ladung gespalten werden können.

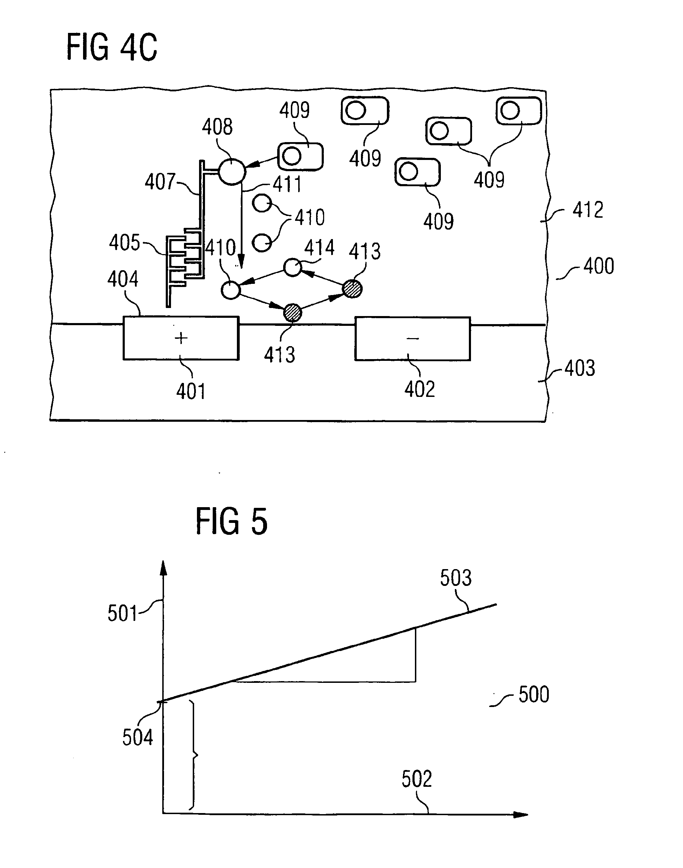

Die negativ geladenen Teilmoleküle werden, wie in **Fig.4c** gezeigt ist, zu der positiv geladenen Anode gezogen, wie durch den Pfeil 411 in **Fig.4c** angedeutet ist.

Die negativ geladenen ersten Teilmoleküle 410 werden an der ersten Elektrode 401, die als Anode ein positives elektrisches Potential aufweist, oxidiert und werden als oxidierte Teilmoleküle 413 an die negativ geladene Katode, d.h. die zweite Elektrode 402 gezogen, wo sie wieder reduziert werden.

Die reduzierten Teilmoleküle 414 wiederum wandern zu der ersten Elektrode 401, d.h. zu der Anode.

Auf diese Weise wird ein elektrischer Kreisstrom generiert, der näherungsweise proportional ist zu der Anzahl der jeweils durch die Enzyme 408 erzeugten Ladungsträger.

Der elektrische Parameter, der bei dieser Methode ausgewertet wird, ist die Änderung des elektrischen Stroms $\dfrac{dI}{dt}$ als Funktion der Zeit t, wie dies in dem Diagramm 500 in **Fig.5.** dargestellt ist.

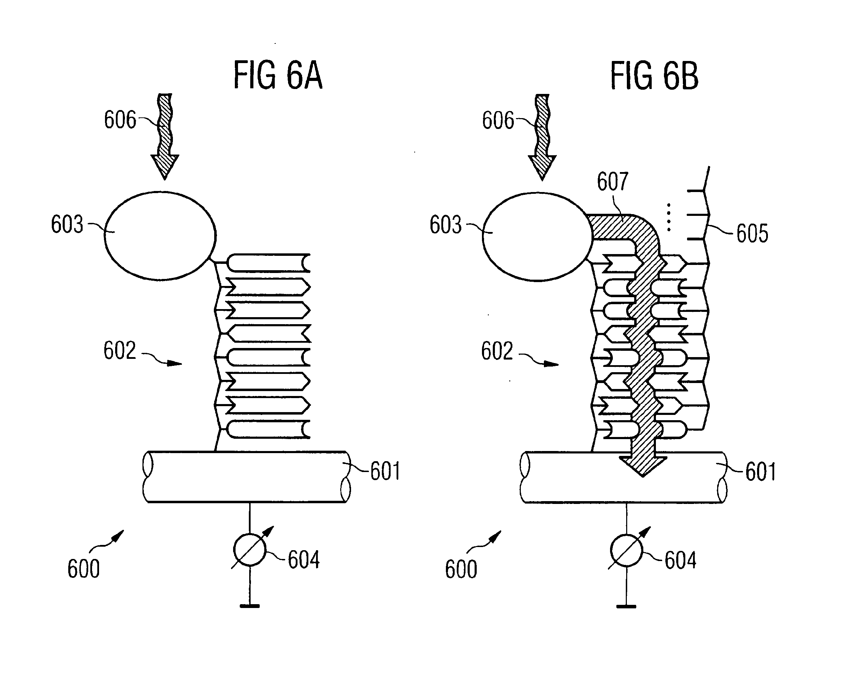

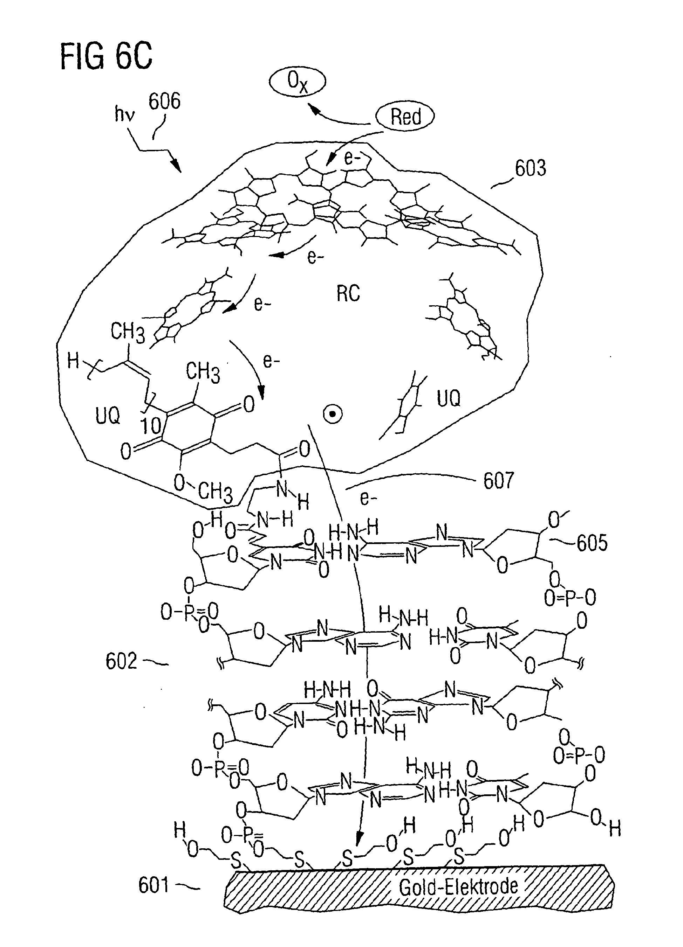

Schließlich ist z.B. aus [6] ein Verfahren bekannt, das auf wie anhand **Fig.6** näher erläutert, auf dem Unterschied der Leitfähigkeit von einzelsträngigen und doppelsträngigen Nukleinsäuremolekülen beruht und deshalb auf eine Messung eines elektrischen Stromes oder einer Spannung zurückgreift.

Bei dem Verfahren nach [6] wird ein einzelsträngiges Nukleinsäuremolekül 602 als Fängermolekül auf einer elektrisch leitfähigen Sensor-Oberfläche 601 immobilisiert. Das Fängermolekül 602 trägt eine Markierung 603, die bei einem durch Licht induzierbaren Redoxvorgang Elektronen abgegeben oder aufnehmen kann.

Wird Licht geeigneter Wellenlänge, wie durch den Pfeil 606 symbolisiert, auf den Sensor 600 gestrahlt, so setzt die redoxaktive Markierung 603, durch das eingestrahlte Licht angeregt, kontinierlich Elektronen frei. Falls die Markierung in einem doppelsträngigen Hybrid aus DNA-Fängermolekül 602 und zu erfassender Nukleinsäure 605 vorliegt, wirkt dieses doppelsträngige Hybrid als eine Art Elektronenpumpe und leitet Elektronen, wie durch den Pfeil 607 veranschaulicht, von der Markierung 603 zur leitfähigen Oberfläche 601, so dass an dieser ein Strom mittels eines Messgeräts 604 gemessen werden kann (vgl. **Fig.6a,b**). Falls jedoch keine Hybridisierung erfolgt, stellen einzelsträngige Fängermoleküle 602 näherungsweise einen Isolator dar, es fließt folglich kein Strom an der Oberfläche 601.

**Fig.6c,** die Fig.4 aus [6] entspricht, zeigt eine im molekularen Detail genauere Darstellung, bei der als redoxaktive Markierung ein photosynthetisches bakterielles Reaktionszentrum verwendet wird.

Die vorstehend genannten Verfahren zur Erfassung von makromolekularen Biopolymeren wie Nukleinsäuren haben den Nachteil gemeinsam, dass ihre Nachweisempfindlichkeit relativ gering ist, insbesondere wenn nur kleine Mengen an nachzuweisenden Moleküle zur Verfügung stehen.

[10] offenbart ein Verfahren und eine Messeinrichtung zur Bestimmung einer Vielzahl von Analyten in einer Probe.

[11] offenbart ein Verfahren zum Erfassen von makromolekularen Biopolymeren mittels einer Elektrodenanordnung.

[12] offenbart ein elektrisches Sensorarray basierend auf voltametrischen und/oder impedimetrischen Detektionsprinzipien mit serieller oder paralleler Auslesung.

[13] offenbart ein integriertes Instrument zum Manipulieren, Durchführen einer Reaktion und Detektieren von Proben in Mikroliter- bis Pikoliter-Dimensionen.

[14] offenbart Strukturen und Verfahren zur Verwendung in der Detektion von Analyten.

[15] offenbart ein Gen-Detektionsverfahren, wobei mittels auf Elektroden oder den Spitzen optischer Fasern immobilisierter Basensequenzen, Gene von Nukleinsäuren detektiert werden.

[16] offenbart ein Verfahren zur Detektion von Nukleinsäure-Oligomer-Hybridisierungsereignissen. Dabei dienen Oligomer-Einzelstränge, die mit einem Ende an einer leitfähigen Oberfläche gebunden sind und mit dem anderen freien Ende mit einer redox-aktiven Einheit verknüpft sind, als Hybridisierungsmatrix.

[17] offenbart eine miniaturisierte integrierte Nukleinsäure-Diagnoseeinrichtung, die einen Bereich zum Extrahieren von Nukleinsäuren und einen Bereich zum Bilden von Bindungen zwischen Nukleinsäuren aufweist.

Der Erfindung liegt das Problem zugrunde, ein Verfahren zur Empfindlichkeitssteigerung sowie eine Vorrichtung für die Erfassung von Nukleinsäuremolekülen bereitzustellen.

Das Problem wird durch die Verfahren sowie den Biosensor mit den Merkmalen gemäß den unabhängigen Patentansprüchen gelöst.

Bei dem ersten Verfahren zum Erfassen von Nukleinsäuremolekülen wird mindestens eine Einheit zum Immobilisieren von Nukleinsäuremolekülen eingesetzt. Diese ist mit ersten Molekülen versehen, die als Fängermoleküle dienen. Bei dem Verfahren wird eine Probe mit der mindestens einen Einheit zum Immobilisieren von makromolekularen Biopolymeren in Kontakt gebracht. Dabei kann die Probe (die vorzugsweise zu erfassenden) Nukleinsäuremoleküle enthalten und die makromolekularen Biopolymere oder die ersten Moleküle weisen dabei eine Markierung auf, mittels der ein detektierbares Signal, beispielsweise ein optisches Signal erzeugt werden kann. In der Probe enthaltene Nukleinsäuremoleküle werden an den Fängermolekülen gebunden, wodurch Komplexe aus Fängermolekülen und Nukleinsäuremolekülen gebildet werden. Anschließend wird mittels der Markierung die Aussendung eines Signals angeregt, beispielsweise Fluoreszenzlicht, dieses mittels der Markierung ausgesandte Signal erfasst, und die Komplexe aus Fängermolekülen und Nukleinsäuremolekülen werden (wieder) beispielsweise mittels thermischer Denaturierung getrennt. Durch diese Trennung wird eine sprunghafte Veränderung der Intensität des ausgesendeten Signals bewirkt. Die Nukleinsäuremoleküle werden dann mittels der Intensität, vorzugsweise mittels der Intensitätsänderung des Signals erfasst.

Bei einem zweiten Verfahren zum Erfassen von Nukleinsäuremolekülen wird eine Elektrodenanordnung verwendet, die mindestens eine Einheit zur Immobilisierung von Nukleinsäuremolekülen aufweist. Für das Verfahren ist die mindestens eine Einheit zum Immobilisieren von Nukleinsäuremolekülen mit ersten Molekülen versehen, die als Fängermoleküle dienen. Eine Probe wird dann mit der mindestens einen Einheit zum Immobilisieren in Kontakt gebracht, wobei die Probe zu erfassende Nukleinsäuremoleküle enthalten kann. Bei dem Verfahren werden in der Probe enthaltene, zu den Fängermolekülen komplementäre Nukleinsäuremoleküle an den Fängermolekülen gebunden. Dadurch werden Komplexe aus Fängermolekülen und Nukleinsäuremolekülen gebildet. Die Änderung des elektrischen Signals während der Komplexbildungsphase (d.h. der Hybridisierungsphase) wird detektiert, wobei insbesondere der Messwert am Ende der Komplexbildungsphase als Ausgangswert festgehalten wird.

Ferner wird bei dem Verfahren eine erste elektrische Messung durchgeführt, vorzugsweise nachdem die mindestens eine Einheit zum Immobilisieren mit der zu untersuchenden Probe in Kontakt gebracht wird. Dann werden die Komplexe aus Fängermolekülen und Nukleinsäuremolekülen, wie z.B. doppelsträngige Hybridmoleküle aus Nukleinsäuren, beispielsweise mittels thermischer Denaturierung getrennt. Dadurch wird eine spontane Veränderung des Wertes der elektrischen Messung bewirkt. Es wird dann oder genau ab der Trennung eine zweite elektrische Messung durchgeführt. Die Nukleinsäuremoleküle werden mittels des Ergebnisses der elektrischen Messung, z.B. anhand der Differenz zwischen dem Wert der ersten Messung und dem Wert der zweiten Messung, erfasst.

Anschaulich ausgedrückt beruhen die Verfahren der Erfindung auf der Erkenntnis, dass die Trennung von makromolekularen Biopolymeren wie z.B. doppelsträngigen Nukleinsäuremolekülen, d.h. das Denaturieren oder Schmelzen von Nukleinsäuremolekülen, ein kooperativer ("sprunghafter") Prozess ist und daher die Denaturierung eine sprunghafte Änderung eines Messwerts oder eines Signals bewirkt, die zum Nachweis von makromolekularen Biopolymeren wie Nukleinsäuren herangezogen werden kann.

Durch die Erfassung gerade dieser sprunghaften Änderung haben die vorliegenden Verfahren gegenüber den bekannten Verfahren den Vorteil einer verbesserten Nachweisempfindlichkeit, insbesondere, wenn nur geringe Mengen an nachzuweisenden Biopolymeren vorhanden sind. Denn es ist gerade bei einem absolut gesehen kleinen Signal genauer, eine einmalige sprunghafte Änderung zu messen als eine kontinuierliche langsame Veränderung, wie sie z.B. bei dem Verfahren aus [4] vonstatten geht, das auf einer Kapazitätsänderung beim Hybridisierungsvorgang beruht.

Im folgenden wird die Erfindung näher erläutert.

Die Denaturierung, d.h. die Trennung der Hybridmoleküle aus Fängermolekülen und zu erfassenden Nukleinsäuremoleküle, kann bei den Verfahren der Erfindung durch sämtliche Maßnahmen erreicht werden, die zu einem kooperativen Bruch der Wasserstoff-Brücken zwischen den jeweils komplementären Nukleotidpaaren, führen. Vorzugsweise wird die Denaturierung durch eine Erhöhung der Temperatur auf eine Temperatur, die oberhalb des Schmelzpunkts (Tm) der doppelsträngigen Hybridmoleküle liegt, bewirkt. Es ist allerdings auch möglich, das Schmelzen der doppelsträngigen Nukleinsäuremoleküle durch Veränderung des pH-Werts, d.h. insbesondere durch Zugabe von Alkali, und/oder durch Zugabe von organischen (aprotischen) Lösungsmitteln wie DMSO oder DMF oder durch die Erhöhung der Ionenstärke (d.h. vorzugsweise durch Erhöhung der Salzkonzentration) zu erzielen. Der für die Durchführung der vorliegenden Verfahren herangezogene Schmelzpunkt Tm kann experimentell bestimmt werden und/oder mit geläufigen Methoden abgeschätzt werden (siehe hierzu z.B. [7]).

Unter Erfassen wird im Sinne der Erfindung sowohl der qualitative als auch quantitative Nachweis von Nukleinsäuremolekülen in einem zu untersuchenden Analyten verstanden. Dies bedeutet, dass der Begriff "Erfassen" ebenfalls einschließt, die Abwesenheit von Biopolymeren Nukleinsäuremolekülen in dem Analyten festzustellen. Im Sinne der Erfindung kann dieser Analyt eine zu untersuchende Probe sein, die zu den immobilisierten Fängermolekülen hinzugegeben wird. Andererseits kann der Analyt jedoch auch die Fängermoleküle enthalten, die dann immobilisiert werden und mit Hilfe der hinzugegebenen makromolekularen Biopolymere erfasst werden.

Zur Ausbildung der doppelsträngigen Hybridmoleküle sind die als Fängermoleküle dienenden ersten Nukleinsäuremoleküle vorzugsweise einzelsträngige Moleküle. Es ist allerdings auch möglich, doppelsträngige Moleküle als Fängermoleküle zu verwenden, die nur einen einzelsträngigen Bereich aufweisen, dessen Sequenz komplementär zu der Nukleotidsequenz der zu erfassenden Nukleinsäuremoleküle ist. Eine weitere Möglichkeit besteht darin, die Bildung des doppelsträngigen Hybrids durch Induktion einer Fluidbewegung senkrecht zu der mindestens einer Einheit zum Immobilisieren, wie z.B. in [8] beschrieben, zu bewirken. In diesem Fall kann das Fängermolekül zunächst auch vollständig als Doppelstrang vorliegen.

Bei dem ersten hier beschriebenen Verfahren wird mittels der Markierung, die sich an den Nukleinsäuremolekülen oder den Fängermolekülen befinden, ein Signal erzeugt.

In einer Ausgestaltung dieses Verfahren ist ein solches Signal ein elektrischer Strom oder eine veränderte Impedanz, insbesondere Kapazität oder Leitfähigkeit im Bereich der Sensorstruktur. In einer weiteren Ausgestaltung ist das Signal sichtbares Licht oder UV-Licht.

Daraus wird ersichtlich, dass bei diesem Verfahren verschiedene Arten der Markierung, die nachfolgend auch als Reportergruppe bezeichnet wird, eingesetzt werden können.

So kann die Markierung eine (chemische) Verbindung oder Gruppe sein, die durch externe Anregung unmittelbar in der Lage ist, ein zur Erfassung der Nukleinsäuremoleküle einsetzbares Signal erzeugen. Beispiele solcher Markierungen sind Fluoreszenz-Farbstoffe (Fluorophore) oder Verbindungen, die eine Chemilumineszenz-Reaktion auslösen können. Eine andere Gruppe solcher Markierungen sind redoxaktive Enzyme, wie sie z.B. in [6] beschrieben sind, und die nach Einstrahlung von Licht geeigneter Wellenlänge einen Elektronenfluss durch doppelsträngige Nukleinsäuremoleküle erzeugen, der gemessen werden kann.

Eine derartige redoxaktive Markierung im Sinne der Erfindung umfasst folglich die in [6] definierten chemisch induzierbaren oder photoinduzierbaren redoxaktiven Einheiten (vgl. die Definition der photoinduzierbar redoxaktiven Einheit in [6] auf Seite 10, 2. Absatz bis Seite 12, 2. Absatz bzw. der chemisch induzierbar redoxaktiven Einheit auf Seite 12, 2. Absatz, Seite 13, 1. Absatz), die (kovalent) über mindestens eine Bindung an ein als Fängermolekül dienendes Nukleinsäure-Einzelstrang-Molekül gebunden sind.

Beispiele für hier einsetzbare photoinduzierbare redoxaktive Markierungen sind folglich das photosynthetische bakterielle Reaktionszentrum (RC), Cyclophane oder ein wenigstens bimolekularer Elektronen-Donor/Elektronen-Akzeptor-Komplex in seiner Bedeutung gemäß [6] (siehe dort Seite 14, 2. Absatz, Seite 15, 1. Absatz).

Beispiele für chemisch induzierbare redoxaktive Markierungen sind daher der Cytochrom-bc-Komplex, der Cytochrom-$c_2$-Komplex der Photosynthese betreibenden Bakterien oder chemisch induzierbare wenigstens bimolekularer Elektronen-Donor/Elektronen-Akzeptor-Komplexe wie geeignete Cyclophane (siehe [6], Seite 31, 2. Absatz).

Angemerkt sei hier, dass die eben beschriebenen redox-aktiven Markierungen zu unterscheiden sind von Markierungen wie Enzyme, die in dem hier ebenfalls anwendbaren Reduktions-/Oxidations-Recycling-Verfahren eingesetzt werden können. Angemerkt sei ferner, dass die redox-aktiven Markierungen bevorzugt in dem Verfahren eingesetzt werden, bei dem die Fängermoleküle eine Markierung tragen.

Zum anderen kann die Markierung eine Substanz sein, die nur mittelbar ein Signal zur Erfassung der Biopolymere wie Nukleinsäuremoleküle erzeugt, d.h. eine Substanz, die die Erzeugung des Signals veranlasst. Eine solche Reportergruppe kann beispielsweise ein Enzym sein, welches eine chemische Reaktion katalysiert, die dann zur Erfassung der Biopolymere herangezogen wird. Beispiele für solche Enzyme sind Alkalische Phosphatase, Glutathion-S-Transferase, Superoxid-Dismutase, Meerrettich-Peroxidase, alpha-Galaktosidase und beta-Galaktosidase. Diese Enzyme sind in der Lage, geeignete Substrate zu spalten, die farbige Endprodukte oder z.B. Verbindungen ergeben, die in dem vorstehend beschriebenen Reduktions-/Oxidations-Recycling-Verfahren eingesetzt werden können. Zu der Gruppe der Markierungen, die nur mittelbar ein zum Nachweis von makromolekularen Biopolymere einsetzbares Signal erzeugen, gehören ferner Liganden für Bindungsproteine und Substrate für Enzyme. Diese Markierungen werden hier allgemein als Enzym-Liganden bezeichnet. Beispiele für solche als Markierung einsetzbare Enzym-Liganden sind Biotin, Streptavidin, Avidin, Digoxigenin oder Substrate für die oben genannten Enzyme.

Diese Unterscheidung zwischen mittelbar und unmittelbar ein Signal erzeugenden Markierung sei anhand der Erzeugung eines Chemilumineszenzsignals im Detail veranschaulicht.

Eine Markierung, die von sich aus (d.h. unmittelbar im vorliegenden Sinne) die Erzeugung von Chemilumineszenzstrahlung bewirken kann, ist z.B. die Meerrettich-Peroxidase, die in der Anwesenheit von Wasserstoffperoxid ($H_2O_2$) die Oxidation von cyclischen Diacylhydraziden wie Luminol katalysiert. Bei dieser chemischen Umsetzung wird ein Reaktionsprodukt in einem angeregten Zustand gebildet, der durch Lichtemission in den Grundzustand übergeht. Diese Lichtemission kann durch weitere chemische Reaktionspartner verstärkt werden, im Falle des Peroxidase-Luminol-Systems z.B. durch 4-Iodphenol. Ein weiteres Beispiel ist die Luciferase aus *Photinus pyralis*, die in Gegenwart von ATP und Sauerstoff die Umwandlung von Luciferin in oxidiertes Luciferin unter Lichtemission katalysiert. Ein anderes Beispiel ist alkalische Phosphatase, die geeignete 1,2-Dioxetane als Substrate verwendet; vgl. [9]. Eine solche Markierung kann, falls gewünscht, direkt mit einem der beiden Bindungspartner (Fängermolekül oder Biopolymer) verknüpft werden.

Es ist jedoch andererseits auch möglich, als Markierung eine chemische Verbindung zu verwenden, die selbst keine Chemilumineszenz-Reaktion auslösen kann, die jedoch spezifische Bindungsaffinität zu einem Bindungspartner hat, der seinerseits z.B. mit einer Enzym wie der Meerrettich-Peroxidase gekoppelt ist. Eine solche Markierung ist z.B. Biotin. Dieses besitzt eine hohe Bindungsaffinität zu dem Protein Streptavidin. Wird Streptavidin z.B. mit der oben genannten Meerrettich-Peroxidase gekoppelt, ist dieses Reagenz in der Lage, einerseits an Biotin, das als Markierung z.B. in ein nachzuweisende Biopolymer eingebaut worden ist, zu binden, und andererseits ein Chemilumineszenz-Reaktion auszulösen. Daher können als Markierung/Markierungskomponente der Fängermoleküle oder zu erfassenden Biopolymere vorliegend auch die oben aufgeführten Verbindungen wie Biotin, Avidin oder Digoxigenin eingesetzt werden. Die Verwendung dieser mittelbar arbeitenden Markierungen kann von Vorteil sein, da sie bildlich gesprochen, am Anfang einer Signalverstärkungskaskade stehen, und deshalb die Nachweisempfindlichkeit des Verfahrens noch steigern können.

Bei dem auf Basis einer Elektrodenanordnung arbeitenden Verfahren (das deshalb keine Markierung wie eben beschrieben benötigt) wird in einer Ausgestaltung bei der elektrischen Messung das an einer Elektrode anliegende Potential gemessen. In anderen Ausgestaltungen wird die Kapazität, der elektrische Widerstand, der elektrische Stromfluss oder die Impedanz zwischen zwei Elektroden bzw. die Veränderung des entsprechenden Werts dieser Messgrößen gemessen.

Diese vorstehend beschriebenen elektrischen Messungen können unter anderem darauf beruhen, dass Nukleinsäuren in Lösung in der Regel Polyanionen sind. Die Denaturierung (der Trennung in die einzelsträngigen immobilisierten Fängermoleküle und die zu erfassenden Nukleinsäuremoleküle) führt daher zu einer diffusionsbedingten Ladungstrennung. Diese entspricht anschaulich ausgedrückt näherungsweise dem Entladevorgang eines geladenen Kondensators, der durch eine geeignete Sensoreinheit nachgewiesen werden kann. Bei dem auf einer Elektrodenanordnung beruhenden Verfahren ist nicht nur vorteilhaft, dass keine vorangehende Markierung der zu erfassenden Nukleinsäuren notwendig ist. Vielmehr ist auch von Vorteil, dass durch die kollektive (kooperative) Ladungsverschiebung z.B. ein vergleichsweise großer Stromfluss oder eine vergleichsweise große Kapazitätsänderung mit einer Elektrode detektiert werden kann. Dies ermöglicht eine hohe Messempfindlichkeit. Deshalb birgt dieses Verfahren insbesondere für eine quantitative Bestimmung der zu erfassenden Nukleinsäuremoleküle Vorteile.

Durch die eben beschriebene Ladungsverschiebung kommt es auch zu einer Änderung des elektrochemischen Potentials, das sich an der Grenzfläche zwischen der Einheit zum Immobilisieren und dem (zu untersuchenden) Medium ausbildet. In einer weiteren Ausführungsform des zweiten Verfahrens wird daher für die Erfassung von Nukleinsäuren dieses elektrische Grenzflächen-Potential gemessen.

In einer Ausgestaltung beider Verfahren, bei der die Denaturierung thermisch erfolgt, geschieht die Temperaturmessung und/oder die Temperaturführung durch eine Thermostatisierungseinheit, die in den Biosensor integriert ist, der die mindestens eine Einheit zum Immobilisieren aufweist. Die Thermostatisierungseinheit kann mit einer externen Steuer- und Kontrolleinheit verbunden sein. Sie kann aber auch selbst alle Kontroll- und Steuereinheiten neben den Heizelementen aufweisen. Im letztgenannten Fall erfolgt die gesamte Temperaturführung deshalb auf dem Chip ("on-chip").

Dies kann beispielsweise mittels Integration von Heizwiderständen, Temperatursensoren und/oder Regelschaltungen realisiert werden.

Die Heizelemente zur Temperierung sollten in gutem thermischem Kontakt zu der Biosensoranordnung stehen. Dies kann wie oben ausgeführt durch eine Integration des Heizwiderstandes auf dem Biochip realisiert werden oder durch einen in das Gehäuse integrierten, außerhalb des Chips angeordneten, sogenannten off-chip Heizwiderstand. Die Temperatursensoren sind bevorzugt in unmittelbarer Nähe zu den Nukleinsäuremolekülen anzubringen um eine exakte und verzögerungsfreie Messung der Temperatur zu ermöglichen. Die Regelschaltung für die Temperaturführung kann als Proportional-, Differential- und/oder Integralregler ausgeführt sein.

Möglich ist selbstverständlich auch eine Temperaturführung durch thermische Kopplung von außen, z.B. durch Widerstände, die in einer Aufnahme- und/oder Haltevorrichtung für den Biosensor integriert sind. Ein Beispiel dafür sind in dem Gehäuse des Biosensors integrierte Widerstände.

Unter "Einheit zur Immobilisierung" wird im Sinne der Erfindung eine Einheit verstanden, die eine Oberfläche aufweist, auf der die als Fängermoleküle dienenden Nukleinsäuremoleküle immobilisiert werden können, d.h. an die die Fängermoleküle durch physikalische oder chemische Wechselwirkungen binden können. Diese Wechselwirkungen schließen hydrophobe oder ionische (elektrostatische) Wechselwirkungen und kovalente Bindungen ein. Beispiele für geeignete Oberflächen-Materialien, die für die mindestens eine Einheit zur Immobilisierung verwendet werden können, sind Metalle wie Gold oder Silber, Kunststoffe wie Polyethylen- oder Polypropylen oder anorganische Stoffe wie Siliziumdioxid.

Ein Beispiel für eine physikalische Wechselwirkung, die eine Immobilisierung der Fängermoleküle bewirkt, ist eine Adsorption an der Oberfläche. Diese Art der Immobilisierung kann beispielsweise stattfinden, wenn das Mittel zur Immobilisierung ein Kunststoffmaterial ist, das für die Herstellung von Mikrotiterplatten verwendet wird (z.B. Polypropylen). Allerdings ist eine kovalente Verknüpfung der Fängermoleküle an die Einheit zum Immobilisieren bevorzugt, weil dadurch die Orientierung der Fängermoleküle gesteuert werden kann. Die kovalente Verknüpfung kann über jede geeignete Verknüpfungschemie ("Linker-Chemie") erfolgen.

Bei einer Ausgestaltung der beiden Verfahren ist die mindestens eine Einheit zum Immobilisieren benachbart zu einer Arbeitselektrode angeordnet, d.h. die Arbeitselektrode ist so in räumlicher Nähe angeordnet, dass sie auf die Einheit oder Einheiten zum Immobilisieren über elektrische Kräfte/Felder einwirken kann. Vorzugsweise ist die Einheit zum Immobilisieren oberhalb der Arbeitselektrode angeordnet. Unter einer Arbeitselektrode wird im Sinne der Erfindung eine Elektrode verstanden, die aktiv bei einem der hier beschriebenen Erfassungsverfahren beteiligt ist, z.B. dadurch dass ein elektrischer Strom gemessen wird, der durch diese Elektrode fließt. Eine Arbeitselektrode im Sinne der Erfindung ist beispielsweise auch eine Elektrode, an die ein negatives Potential angelegt wird, welches die Trennung eines doppelsträngigen Hybridmoleküls in die beiden negativ geladenen Einzelstränge fördert.

Zu unterscheiden ist eine solche Arbeitselektrode von einer Kontrollelektrode. Eine Kontrollelektrode ist eine Elektrode, die nicht aktiv an der Erfassung von Nukleinsäuren beteiligt ist. Wird beispielsweise mit dem auf einer Elektrodenanordnung basierenden Verfahren eine Impedanzmessung durchgeführt, kann eine der dazu notwendigen zwei Elektroden ausschließlich als Kontrollelektrode dienen. Das ist z.B. der Fall, wenn das Potential an der Grenzfläche zwischen der Einheit zum Immobilisieren und einem zu untersuchenden Medium zur Erfassung herangezogen wird.

Bei dem ersten der zwei hier offenbarten Verfahren, das mit einer Markierung arbeitet, kann auf eine Referenzelektrode im vorstehenden Sinn gänzlich verzichtet werden, wenn z.B. eine optische Erfassung des Messsignals durch eine Floureszenz- oder Chemilineszenzmarkierung erfolgt.

An dieser Stelle sei zusammenfassend gesagt, dass eine Kontrollelektrode nur dann notwendig ist, wenn absolute elektrische Potentiale an den Arbeitselektroden angelegt oder gemessen werden sollen. Die Kontrollelektrode, deren Potential stromfrei gemessen wird, liefert hier das Bezugspotential. In reinen elektrochemischen Verfahren (z.B. Redox-Recycling) ist eine derartige Elektrode zur Messung absolut notwendig. Nicht notwendig ist eine Kontrollelektrode hingegen, wenn der Einsatz elektrischer Felder lediglich zur Förderung der Denaturierungsreaktion erfolgt. In diesem Falle ist die exakte Kenntnis des Elektrodenpotentials nicht zwingend notwendig. Im diesen Zusammenhang sei angemerkt, dass eine solche zur Förderung der Komplexbildung oder Denaturierung verwendete Elektrode z.B. auch bei optischer Erfassung des Signals eingesetzt werden kann.

In einer weiteren Ausführungsform der Verfahren ist die mindestens eine Einheit zum Immobilisieren direkt auf einer Arbeitselektrode aufgebracht. In einer weiteren Ausgestaltung der Verfahren ist die mindestens eine Einheit auf einer Photodiode aufgebracht.

In einer anderen Ausgestaltung ist die mindestens eine Einheit zum Immobilisieren als Arbeitselektrode ausgestaltet.

Wie bereits oben erwähnt, kann bei den hier beschriebenen Verfahren an die Arbeitselektrode ein negatives elektrisches Potential zur Trennung der Hybridmoleküle angelegt werden. Dadurch wird die Kollektivität des Denaturierungsvorgangs noch verstärkt und somit die Messgenauigkeit und/oder Messempfindlichkeit weiter verbessert.

In einer weiteren Ausführungsform der beiden Verfahren kann an eine oder mehrere Arbeitselektroden ein positives elektrisches Potential angelegt werden, entweder bevor oder nachdem die Fängermoleküle mit dem zu untersuchenden Medium in Kontakt gebracht worden sind. Durch dieses positive elektrische Potential kann der normalerweise diffusionsgetriebene Hybridisierungsvorgang beschleunigt werden, indem die negative geladenen Nukleinsäuremoleküle durch geeignete elektrische Felder an die mindestens eine Einheit zum Immobilisieren gezogen werden. Es ist natürlich in Abhängigkeit vom jeweiligen Sensoraufbau auch möglich, mehrere Elektroden für diesen Zweck einzusetzen. Ferner ist es selbstverständlich auch möglich, diejenige Arbeitselektrode oder -elektroden, die für zur Beschleunigung des Schmelzvorgangs eingesetzt werden, auch zur Beschleunigung des Hybridisierungsvorgang zu verwenden.

Bei beiden hier offenbarten Verfahren können mehrere Einheiten zum Immobilisieren von Nukleinsäuren verwendet werden, wobei diese Einheiten in einer regelmäßigen Anordnung, einem Array, angeordnet sein können.

Der hier beschriebene Biosensor zum Erfassen von Nukleinsäuremolekülen weist mindestens eine Einheit zum Immobilisieren von Nukleinsäuren, ein Array von Erfassungseinheiten sowie eine Mehrzahl von in den Sensor integrierten Thermostatisierungseinheiten auf. Die Erfassungseinheiten sind derart ausgestaltet, dass Nukleinsäuremoleküle, die an auf der Einheit zum Immobilisieren aufgebrachte Fängermoleküle gebunden sind, mittels der Erfassungseinheiten erfasst werden.

Eine Erfassungseinheit im Sinne der Erfindung ist eine Einheit, die ein Signal, das von einer Markierung an den Nukleinsäuremolekülen ausgesandt wird, bzw. die eine elektrische Messgröße, die durch die Anwesenheit oder Abwesenheit von (doppelsträngigen) Nukleinsäuremolekülen, beeinflusst wird, erfassen und weiterleiten kann. Beispiele für eine solche Erfassungseinheit sind eine Photodiode, eine Elektrodenpaar oder ein MOSFET. Alternativ kann die Erfassungseinheit jedoch auch off-chip realisiert sein, beispielsweise als optischer Detektor (CCD-Kamera) im Auslese-/Betriebsgerät für den erfindungsgemäßen Sensor.

7

Der Begriff "in den Sensor integriert" ist hier so zu verstehen, dass die Thermostatisierungseinheit Bestandteil des eigentlichen Sensors ist. Dies bedeutet, dass die Thermostatierungseinheit nicht in einer externen Aufnahme- oder Haltevorrichtung wie einem Gehäuse für den Biosensor untergebracht ist.

Die in dem Sensor integrierte Thermostatisierungseinheit dient zur Denaturierung und/oder Hybridisierung und/ oder Steuerung des Bindungsverhaltens (Anziehung, Bindung oder Abstoßung von Molekülen, beispielsweise eine thermische Steuerung der Linker-Chemie) der doppelsträngigen

Hybridmoleküle. Sie kann z.B. herkömmlichen Heizelemente wie elektrische Heizwiderstände aufweisen sowie die für die Kontrolle der Temperatur notwendigen Steuer- und Messeinheiten. Weiterhin kann die Thermostatisierungseinheit verwendet werden zur Synthese und/oder Vervielfältigung beispielsweise über Polymerase-Kettenreaktion (PCR). Ferner kann die Thermostatisierungseinheit zur Beschleunigung des Reaktionsvorgangs eingesetzt werden, indem durch die Temperatur die Konvektion und damit die Diffusionsgeschwindigkeit im Reaktionsraum gesteuert werden kann.

In einer Weiterbildung weist der Biosensor zumindest eine Arbeitselektrode auf. Diese ist benachbart zu der mindestens einen Einheit zum Immobilisieren angeordnet, so dass die Einheit von einem elektrischen Potential oder Feld dieser Arbeitselektrode beeinflusst wird. Auf diese Weise kann die vorstehend beschriebene Beschleunigung der Bildung der Hybridmoleküle bzw. des Denaturierungsvorgangs durchgeführt werden. Vorzugsweise ist die mindestens eine Einheit zum Immobilisieren oberhalb der Arbeitselektrode angeordnet.

In einer anderen Ausgestaltung des Biosensors ist die mindestens eine Einheit zum Immobilisieren auf einer Arbeitselektrode oder einer Photodiode aufgebracht. Die Einheit zum Immobilisieren kann z.B. auf einem CMOS-Chip aufgebracht sein. Im Falle einer optisch auswertbaren Markierung kann die mindestens eine Einheit zum Immobilisieren auch auf einer CMOS-Kamera oder einem CCD aufgebracht sein.

In einer weiteren Ausgestaltung des Biosensors ist die mindestens eine Einheit zum Immobilisieren als Arbeitselektrode ausgestaltet.

In einer weiteren Ausführungsform weist die Vorrichtung mehrere Einheiten zum Immobilisieren von makromolekularen Biopolymeren in einer regelmäßigen Anordnung (einem Array) auf.

Unter Nukleinsäuremolekülen werden in diesem Zusammenhang beispielsweise (längerkettige) DNA-Moleküle und RNA-Moleküle, PNA-Moleküle, cDNA-Moleküle, oder auch kürzere Oligonukleotide mit beispielsweise 10 bis 50 Basenpaaren (bp), insbesondere 10 bis 30 Basenpaaren, verstanden. Die Nukleinsäuren können doppelsträngig sein, jedoch auch zumindest einzelsträngige Bereiche aufweisen oder, zum Beispiel durch vorangehende thermische Denaturierung (Strangtrennung) für ihren Nachweis, als Einzelstränge vorliegen. Die Sequenz der zu erfassenden Nukleinsäuren kann dabei zumindest teilweise oder vollständig vorgegeben, d.h. bekannt sein.

Wenn Nukleinsäuremoleküle einer vorgegeben Nukleotidsequenz mit dem hier beschriebenen Verfahren erfasst werden, so werden sie vorzugsweise in einzelsträngiger Form erfasst, d.h. sie werden ggf. vor der Erfassung durch Denaturierung in Einzelstränge überführt. In diesem Fall werden als Fängermoleküle Nukleinsäuremoleküle mit einer zu dem einzelsträngigen Bereich komplementären Sequenz verwendet. Diese Nukleinsäure-Fängermoleküle können wiederum Nukleinsäuremoleküle mit ca. 20 bp bis ca. 50 bp sein oder auch längere Nukleotidsequenzen mit bis zu ca. 500 bp oder länger besitzen, solange sie keine intermolekularen Strukturen ausbilden, die eine Hybridisierung des Fängermoleküls mit der zu erfassenden Nukleinsäure verhindern.

Mit den vorliegenden Verfahren ist es selbstverständlich möglich, nicht nur eine einzige Art von Nukleinsäuremolekülen in einer einzelnen Messreihe zu erfassen. Vielmehr können mehrere Nukleinsäuremoleküle gleichzeitig oder auch nacheinander erfasst werden. Dazu können auf der Einheit zum Immobilisieren mehrere Arten von Fängermolekülen, von denen jedes eine (spezifische) Bindungsaffinität für ein bestimmtes zu erfassendes Nukleinsäuremolekül aufweist, gebunden werden, und/oder es können mehrere Einheiten zum Immobilisieren eingesetzt werden, wobei an jeder von diesen Einheiten nur eine Art von Fängermolekül gebunden wird. Bei diesen Mehrfachbestimmungen wird, wenn Markierungen zum Nachweis verwendet werden, für jedes zu erfassende Nukleinsäuremolekül vorzugsweise eine von den anderen Markierungen unterscheidbare Markierung verwendet. Beispielsweise können zwei oder mehrere Fluorophore als Markierungen verwendet werden, wobei jedes dieser Fluorophore vorzugsweise eine spezifische Anregungs- und Emissionswellenlänge besitzt.

Bei den hier offenbarten Verfahren wird in einem ersten Verfahrensschritt jeweils die zumindest eine Einheit zum Immobilisieren mit den Fängermolekülen versehen.

Eine zu untersuchende Probe, vorzugsweise ein flüssiges Medium wie ein Elektrolyt, wird dann mit der Einheit zum Immobilisieren in Kontakt gebracht. Dies erfolgt in der Weise, dass die Nukleinsäuremoleküle an die Fängermoleküle binden können, d.h. bei einer Temperatur, die unterhalb des Schmelzpunktes der doppelsträngigen Hybridmoleküle liegt. Für den Fall, dass sich in dem Medium mehrere zu erfassende Nukleinsäuren befinden, werden die Bedingungen so gewählt, dass diese dabei jeweils zur gleichen Zeit oder nacheinander an ihre entsprechendes Fängermolekül zur Ausbildung der doppelsträngigen Hybridmoleküle binden können.

Anschließend können nicht hybridisierte zu erfassende Nukleinsäuremoleküle aus dem Reaktionsraum durch einen geeigneten Waschschritt entfernt werden.

Bei dem ersten Verfahren wird danach zur Erfassung der Nukleinsäuren die entsprechende Markierung zur Aussendung eines Signals angeregt und das Signal detektiert. Dazu wird z.B. ein durch das vorstehend beschriebene Redox-Recycling verursachte elektrische Strom oder ausgesandte Fluoreszenzstrahlung gemessen.

Es kann eine einzige Referenzmessung durchgeführt werden, allerdings ist es zweckmäßiger, eine kontinuierliche Messung/Erfassung des ausgesandten Signals bis zum Ende des Verfahrens durchzuführen. Zu Beginn der Messung (oder auch früher oder später) wird die Temperatur in dem Reaktionsraum, d.h. zumindest an der Oberfläche der mindestens einen Einheit zum Immobilisieren, bis zu einer Temperatur erhöht, die oberhalb des Schmelzpunktes der doppelsträngigen Hybridmoleküle liegt. Die Denaturierung kann z.B. auch durch eine impulsartige Veränderung des pH-Werts erzielt werden. Wird der Schmelzpunkt erreicht, so verändert sich die Intensität des Signals durch die Denaturierung schlagartig, sprich, es kommt idealerweise zu einem Abfall auf Null, da die zu erfassenden Nukleinsäuremoleküle nun wieder frei in Lösung vorliegen und durch Diffusion aus dem beobachteten Reaktionsraum entfernt werden. Das Entfernen der einzelsträngigen Moleküle von der Oberfläche der Einheit zum Immobilisieren kann durch Anlegen eines Konvektionsstroms verstärkt und beschleunigt werden.

Diese Signalveränderung wird vorliegend zur Erfassung herangezogen.

Bei dem zweiten Verfahren der Erfindung werden prinzipiell dieselben Verfahrensschritte durchgeführt.

Bei dem zweiten Verfahren können verschiedene Elektrodenanordnungen verwendet werden, die mindestens eine Einheit zum Immobilisieren aufweisen. Beispiele für hier einsetzbare Elektrodenanordnungen sind elektrisch leitfähige Gitter oder Netze, elektrisch leitfähige poröse Materialien, Plattenelektrodenanordnung oder eine Interdigitalelektrodenanordnung, wie aus [1] bekannt. Bei einer Interdigitalelektrodenanordnung können mehrere oder alle "Finger" der Anordnung mit Einheiten zum Immobilisieren versehen sein bzw. selbst als diese Einheiten ausgestaltet sein.

Ferner können verschiedene Anordnungen der Parallelschaltung von Elektroden in der Elektrodenanordnung vorgesehen sein, beispielsweise können die Elektroden als zylindrische Elemente ausgestaltet sein, die jeweils konzentrisch umeinander angeordnet sind und elektrisch voneinander beispielsweise mittels eines geeigneten Dielektrikums voneinander isoliert sind, so dass sich ein elektrisches Feld zwischen den Elektroden ausbildet.

In einem bevorzugten Messverfahren werden mit dem erfindungsgemäßen Sensor mehrere Hybridisierungs/ Denaturierungszyklen durchgeführt. Die dadurch periodisch stattfindende spontane Signaländerung kann zu einer weiteren Erhöhung der Empfindlichkeit und Messgenauigkeit ausgenutzt werden, indem die Messergebnisse mehrerer Zyklen aufgezeichnet und anschließend statistisch ausgewertet, beispielsweise kann der statistische Mittelwert gebildet werden.

Ausführungsbeispiele der Erfindung sind in den Figuren dargestellt und werden im weiteren näher erläutert.

Es zeigen

Figuren 1a und 1b      eine Ausführungsform eines Biosensors gemäß der Erfindung sowie diesen Biosensor zu unterschiedlichen Verfahrenszuständen bei einem der hier offenbarten Verfahren;

Figuren 2a und 2b      eine Skizze zweier Planarelektroden, mittels derer die Existenz zu erfassender DNA-Stränge in einem Elektrolyt (Figur 2a) bzw. deren Nichtexistenz (Figur 2b) nachgewiesen werden können;

Figuren 3a und 3b      weitere Ausführungsformen eines Biosensors gemäß der Erfindung, mit denen weitere Ausführungsarten der hier beschriebenen Verfahren durchgeführt werden können;

Figuren 4a bis 4c      Skizzen eines Biosensors gemäß dem Stand der Technik, anhand derer einzelne Zustände im Rahmen des Redox-Recycling-Vorgangs erläutert werden;

Figur 5      einen Funktionsverlauf eines Kreisstroms gemäß dem Stand der Technik im Rahmen eines Redox-Recycling- Vorgangs;

Figuren 6a bis 6c      das aus [3] bekannte Verfahren für die Erfassung von Nukleinsäuren;

Figur 7      einen Biosensor, anhand dessen eine weitere Ausführungsart eines Verfahrens der Erfindung veranschaulicht wird.

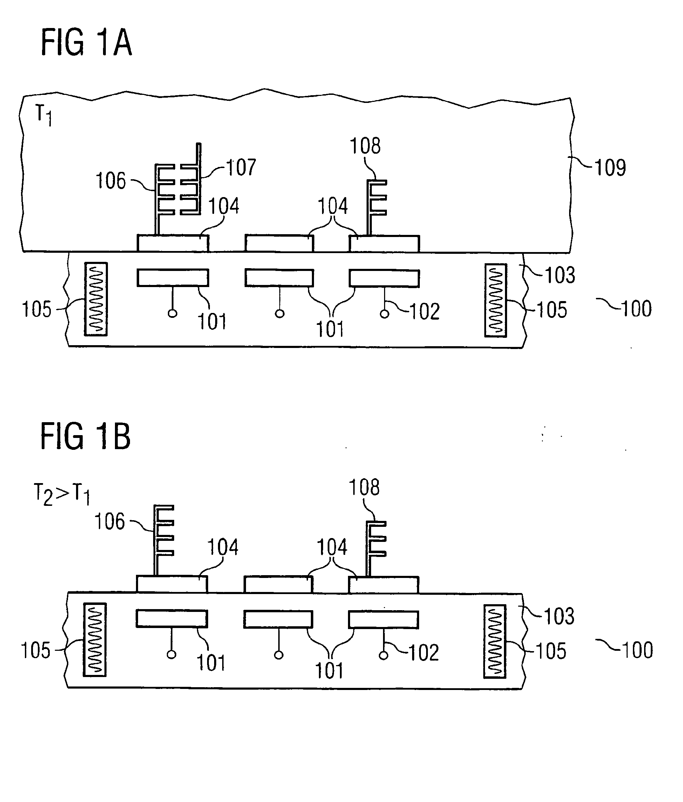

**Fig.1** zeigt einen Ausschnitt aus einem Biosensors 100, mit dem ein erstes Ausführungsbeispiel des hier beschriebenen Verfahrens durchgeführt werden kann.

**Fig.1a** zeigt den Biosensor 100 mit mehreren Elektroden 101, die jeweils über einen elektrischen Anschluss 102 mit einer nicht dargestellten Auswerteeinheit verbunden sind. Die Elektroden sind in eine Isolatorschicht 103 aus Isolatormaterial eingebettet. Auf der Isolatorschicht und oberhalb der Elektroden 101 sind Einheiten 104 zum Immobilisieren von Nukleinsäuren aufgebracht. Der Biosensor 100 weist weiterhin, in das "Sensorsubstrat" eingebettet, eine Thermostatisierungseinheit 105 auf, die zur Temperaturführung dient.

Der Sensor 100 ist ein Beispiel eines Sensors, bei dem die Elektroden lediglich zur Detektion der Nukleinsäuremoleküle anhand einer durch das Schmelzen der doppelsträngigen Hybridmoleküle induzierten Änderung eines elektrischen Feldes dienen.

Die Einheiten zum Immobilisieren 104 des Sensors 100 sind aus Gold hergestellt.

Alternativ können die Einheiten 104 zum Immobilisieren auch aus Siliziumoxid hergestellt sein und mit einem Material beschichtet werden, das geeignet ist, Fängermoleküle zu immobilisieren.

Beispielsweise können bekannte Alkoxysilanderivate verwendet werden wie

- 3-Glycidoxypropylmethyloxysilan,
- 3-Acetoxypropyltrimethoxysilan,
- 3-Aminopropyltriethoxysilan,
- 4-(Hydroxybutyramido)propyltriethoxysilan,
- 3-N,N-bis(2-hydroxyethyl)aminopropyltriethoxysilan, oder andere artverwandte Materialien, die imstande sind, mit einem ihrer Termini eine Bindung, beispielsweise eine kovalente Bindung, mit der Oberfläche des Siliziumoxids einzugehen und mit ihrem anderen Terminus dem zu immobilisierenden Nukleinsäuremoleküls eine chemisch reaktive Gruppe wie einen Epoxy-, Acetoxy-, Amin- oder Hydroxylrest zur Reaktion anzubieten.

Alternativ kann beispielsweise Poly-L-Lysin verwendet werden.

Reagiert ein zu immobilisierendes Fängermolekül mit einer solchen aktivierten Gruppe, so wird es über das gewählte Material als eine Art kovalenter Linker auf der Oberfläche der Beschichtung auf der Einheit zum Immobilisieren gebunden.

Auf den Einheiten zum Immobilisieren 104 werden DNA-Sondenmoleküle 106 und 108 als Fängermoleküle aufgebracht.

Dabei besitzen die ersten DNA-Sondenmoleküle 106 eine zu einer vorgegebenen ersten DNA-Sequenz komplementäre Sequenz. Die zweiten Fängermoleküle 108 besitzen eine Nukleotidsequenz, die zu einer zweiten vorgegebenen DNA-Sequenz komplementär ist. Die Immobilisierung der Fängermoleküle 106 und 108 erfolgt über die sogenannte Gold-Schwefel-Kopplung.

An die Purinbasen Adenin (A), Guanin (G), und die Pyrimidinbasen Thymin (T) oder Cytosin (C) können jeweils zu den Sequenzen der Sondenmoleküle komplementäre Sequenzen der DNA-Stränge in der üblichen Weise, d.h. durch Basenpaarung über Wasserstoffbrückenbindungen zwischen A und T bzw. zwischen C und G, hybridisieren. Bei Verwendung anderer Nukleinsäuremoleküle werden entsprechend andere Basen, im Falle eines RNA-Moleküls beispielsweise Uridin (U), verwendet.

**Fig.1a** zeigt ferner einen Elektrolyten 109, der mit den Einheiten zum Immobilisieren 104 und den DNA-Sondenmolekülen 106 und 108 bei einer Temperatur T1 in Kontakt gebracht wird. Die Temperatur T1 liegt dabei unterhalb des Schmelzpunkts Tm der doppelsträngigen Hybridmoleküle aus den Fängermolekülen 106 bzw. 108 mit ihren zu erfassenden Nukleinsäuremolekülen. Die unter den Versuchsbedingungen gegebene Schmelztemperatur Tm wird gegebenenfalls zuvor abgeschätzt oder experimentell bestimmt.

**Fig.1a** zeigt den Biosensor 100 für den Fall, dass in dem Elektrolyten 109 DNA-Moleküle 107 enthalten sind, die eine vorgegebene erste Nukleotidsequenz aufweisen, die komplementär zu der Sequenz der ersten DNA-Sondenmoleküle 106 ist. In diesem Fall hybridisieren die zu den ersten DNA-Sondenmolekülen 106 komplementären DNA-Stränge 107 mit den ersten DNA-Sondenmolekülen 106, die auf den Einheiten 104 aufgebracht sind.

Da die Sequenzen von DNA-Strängen nur mit der jeweils spezifischen Komplementärsequenz hybridisieren, hybridisieren die zu den ersten DNA-Sondenmolekülen komplementären DNA-Stränge nicht mit den zweiten DNA-Sondenmolekülen 108.

Vor oder aber auch nach der Hybridisierung wird an den Elektroden 102 eine erste elektrische Messung durchgeführt. Bei dieser Ausführungsform wird vorzugsweise das an den Elektroden anliegende Potential gemessen.

Das Resultat nach erfolgter Hybridisierung ist, dass sich auf einer der Einheiten 104 doppelsträngige Hybridmoleküle befinden, d.h. doppelsträngige DNA-Moleküle immobilisiert sind. Auf der anderen Einheit 104 sind nur die zweiten DNA-Sondenmoleküle 108 als weiterhin einsträngige Moleküle vorhanden.

Nach einem optionalen Waschschritt wird die Temperatur mittels der Thermostatisierungseinheit 105 kontinuierlich erhöht. Dabei wird die Schmelztemperatur Tm der Hybridmoleküle aus den Fängermolekülen 106 und den zu erfassenden Molekülen 107 erreicht. Zu diesem Zeitpunkt erfolgt die Denaturierung der Doppelstrang-Moleküle, d.h. die Trennung in die beiden Einzelstränge 106 und 107. Diese Dissoziation bewirkt aufgrund des polyanionischen Charakters der Nukleinsäuren eine sprunghafte Ladungstrennung, die durch eine zweite Potentialmessung an den Elektroden 102 detektiert wird. Durch den Vergleich der für das Potential bei den zwei Messungen erhaltenen Werte wird die Anwesenheit bzw. Abwesenheit der DNA-Moleküle 107 ermittelt. Übersteigt die ermittelte Differenz einen (vorgegebenen) Schwellenwert, so wird daraus geschlossen, dass die DNA-Moleküle 107 in der Probe vorhanden waren und ggf.

in welcher Konzentration. Bei einer Differenz unterhalb des Schwellenwertes wird daraus geschlossen, dass keine DNA-Moleküle 107 vorhanden waren. Diese Klassifizierung nach Schwellenwert kann auch bei den anderen hier offenbarten Verfahren durchgeführt werden.

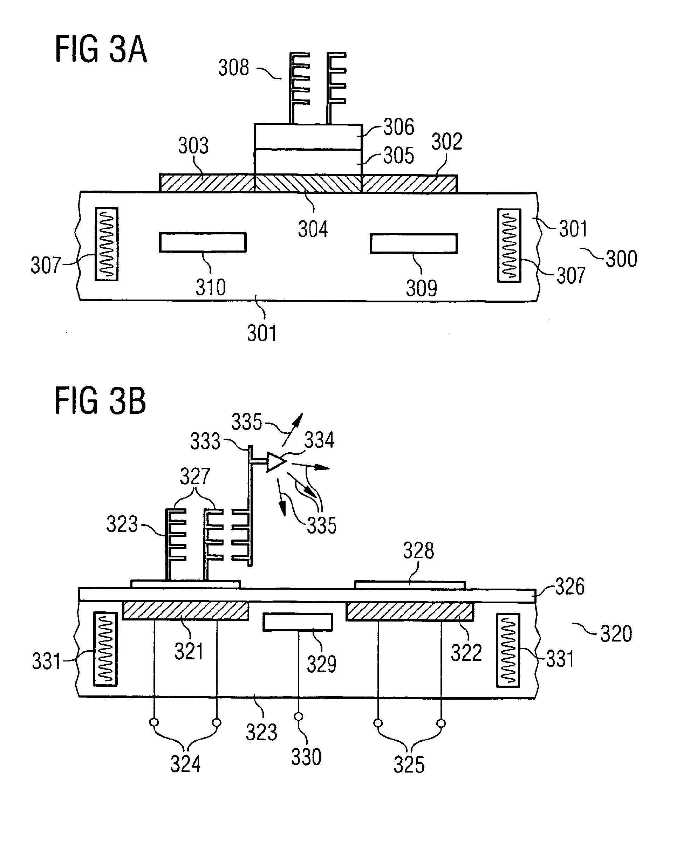

**Fig.3a** zeigt einen Biosensor 300, der mit einem MOSFET für die Erfassung der Nukleinsäuremoleküle versehen ist.

Der Biosensor 300 weist ein Substratmaterial 301 auf, auf dem sich (zum Beispiel n- oder p-halbleitende) Schichten 302 und 303 als Source bzw. Drain eines MOSFETs befinden. Ferner weist der Sensor eine als Kanalbereich dienende Schicht 304 auf. Auf dem Kanalbereich 304 befindet sich die Oxidschicht 305 des MOSFETs. Auf der Oxidschicht 305 ist ein metallisches Gate 306 aufgebracht. Dieses ist aus Gold hergestellt und dient als Einheit zum Immobilisieren von Nukleinsäuren. Ferner weist der Sensor eine Einheit 307 für die Temperaturführung auf. Auf dem Gate 306 sind, zum Beispiel über Gold-Schwefel-Kopplung, Fängermoleküle 308 immobilisiert. Die elektrischen Anschlüsse von Source, Drain und Gate des MOSFETs sind nicht gezeigt. Der Sensor 300 kann (optional) ferner weitere Elektroden 309 und 310 benachbart zu dem MOSFET aufweisen. Die Elektroden 309 und 310 dienen nicht zur Detektion der Nukleinsäuremoleküle, sondern mit ihnen werden elektrische Felder erzeugt, um den Hybridisierungs- und Denaturierungsvorgang zu fördern.

Mit Hilfe des Biosensors 300 kann das anhand Fig.1 beschriebene Verfahren zum Erfassen von Nukleinsäuremolekülen durchgeführt werden. Dabei dient das Gate 306 nicht nur als Einheit zum Immobilisieren, sondern auch als "aktive" Elektrode, d.h. Arbeitselektrode im Sinne der Erfindung. Bei dem Biosensor 300 wird durch die aufgrund der Hybridbildung immobilisierten Ladungen ein elektrisches Feld erzeugt, das den Kanalstrom des MOSFETs moduliert. Somit stellt der Strom durch den Kanal, der sich z.B. durch die Denaturierung ändert, ein direktes Maß für die auf der Einheit 306 zum Immobilisieren befindliche Ladungsmenge dar. Die Veränderung dieses Kanalstroms wird folglich zum Erfassen der Nukleinsäuremoleküle herangezogen.

**Fig.3b** zeigt einen weiteren Biosensor 320 der Erfindung, mit dem ein weiteres hier beschriebenes Verfahren ausgeführt werden kann.

Der Biosensor 320 weist eine erste Fotodiode 321 und eine zweite Fotodiode 322 auf, die in eine Isolatorschicht 323 aus Isolatormaterial wie Silizium eingebracht sind. Die Fotodioden 321, 322 sind über erste und zweite elektrische Anschlüsse 324 und 325 mit einer nicht dargestellten Auswerteeinheit verbunden.

Der Biosensor 320 weist weiterhin eine Oxidschicht 326 auf, auf der sich die Einheiten 327 und 328 zum Immobilisieren von Nukleinsäuren befinden. Diese Einheiten 327, 328 zum Immobilisieren sind aus Gold ausgebildet. Alternativ können die Einheiten 306 zum Immobilisieren auch aus Siliziumoxid hergestellt sein und mit einem z.B. oben genannten Material beschichtet werden, das geeignet ist, Fängermoleküle zu immobilisieren.

Ferner weist der Biosensor 320 benachbart zu den Einheiten 327 und 328 eine Elektrode 329 mit einem elektrischen Anschluss 330 auf. An die Elektrode 329 kann ein elektrisches Potential angelegt werden und dadurch der Hybridisierungs- und/oder der Denaturierungsvorgang beschleunigt werden.

Schließlich befindet sich in dem Sensor 320 eine Thermostatisierungseinheit 331.

Auf der Einheit 327 zum Immobilisieren sind Fängermoleküle 332 immobilisiert. **Fig.3b** zeigt den Sensor in dem Zustand, dass zu erfassende Nukleinsäuremoleküle 333 mit den Fängermolekülen 332 doppelsträngige Hybridmoleküle ausgebildet haben. Die Nukleinsäuremoleküle 333 tragen ein Fluorophor 334 als Markierung, die Fluoreszenzstrahlung emittieren kann. Diese durch die Pfeile 335 angedeutete Strahlung kann von der Fotodiode 321 detektiert werden.

Als ein weiteres Ausführungsbeispiel für ein hier offenbartes Verfahren sei das Redox-Recycling Verfahren mit der bekannten Elektroden-Anordnung gemäß **Fig.4** genannt. Bei dieser Ausführungsform wird nach der erfolgten Ausbildung der doppelsträngigen Hybridmoleküle (vgl. **Fig.4c**) mittels des Enzyms 408 das Molekül 409 gespalten und dadurch der vorstehend beschriebene Redox-Recycling-Vorgang initiiert. Durch eine Temperaturerhöhung über den Schmelzpunkt des Hybrids aus Fängermolekül 405 und zu erfassender Nukleinsäure 407 wird das mit dem Nukleinsäuremolekül 407 verbundene Enzym 408 von der Elektrodenoberfläche entfernt und somit aus dem Molekül 409 dort keine neuen Redox-Paare gebildet, die zum Stromfluß beitragen können. Diese spontane Änderung in der Leitfähigkeit zwischen beispielsweise interdigitalen Elektrodenstrukturen wird zur Erfassung der Nukleinsäuren verwendet.

In einer weiteren alternativen Ausführungsform können ein ein- oder mehrdimensionales Array von Erfassungseinheiten vorgesehen sein und eine Mehrzahl von Thermostatisierungseinheiten. Zumindest unter einem Teil der Erfassungseinheiten, vorzugsweise unter jeder Erfassungseinheit ist jeweils eine Thermostatisierungseinheit angeordnet. Jede Thermostatisierungseinheit ist individuell ansteuerbar und regelbar, so dass jeder Erfassungseinheit lokal erhitzt werden kann.

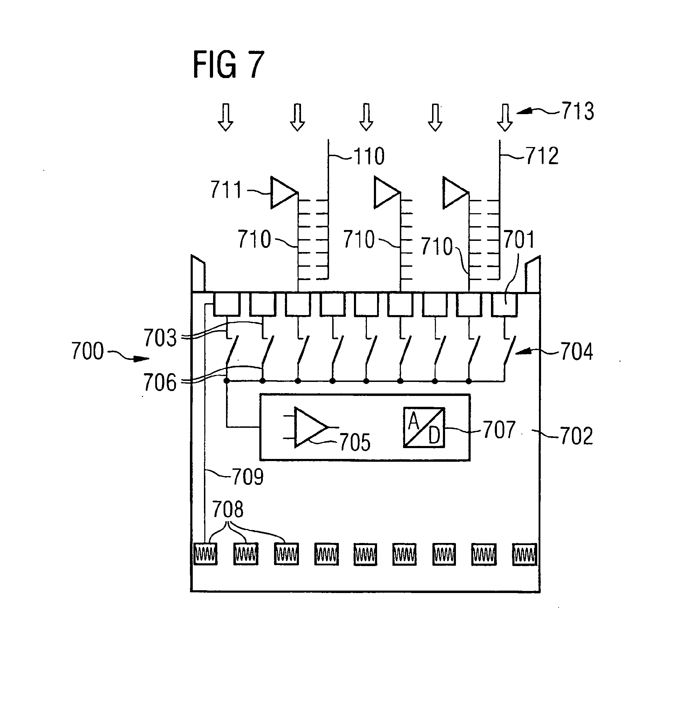

**Fig.7** zeigt einen Biosensor 700 mit Einheiten zum Immobilisieren 701, die in einer Isolatorschicht 702 aus Isolatormaterial angeordnet sind.

Die Einheiten zum Immobilisieren 701 sind über elektrische Anschlüsse 703 mit einer elektrischen Erfassungsschaltung 704, die Teil der Erfassungseinheit des Sensors sind, verbunden. Die Einheiten zum Immobilisieren 701 sind aus Gold hergestellt.

Die elektrische Erfassungsschaltung 704 des Biosensors 700 weist einen Vorverstärker 705 für die Verstärkung des erfassten Signals für jede Einheit zum Immobilisieren, eine Auswahlelektronik 706 für das individuelle Auswählen von mindestens einer Einheit zum Immobilisieren sowie einen Analog/Digital-Wandler 707 für die Umwandlung des erfassten Signals für jede Einheit zum Immobilisieren auf.

Des weiteren weist der Biosensor Thermostatisierungseinheiten 708 auf, die die Temperatur von jeder der Einheiten 701 z.B. über einen elektrische Verbindung 709 individuell ansteuern und regeln können. Der Übersichtlichkeit halber ist eine solche Verbindung 709 nur für eine Thermostatisierungseinheit 708 gezeigt.

Auf den Einheiten zum Immobilisieren 701 sind einzelsträngige DNA-Sondenmoleküle 710 aufgebracht, die eine redoxaktive Markierung 711 aufweisen. Diese Markierung 711 kann z.B. ein photosynthetisches bakterielles Reaktionszentrum sein und wie in [6] auf Seiten 50, 4. Absatz bis Seite 52, 1. Absatz beschrieben, mit den Sondenmolekülen verknüpft werden (vgl. auch **Fig.6c**).

Zur Erfassung von Nukleinsäuren wird der Biosensor 700 in Kontakt mit einer zu untersuchenden Probe, etwa einem Elektrolyten (nicht gezeigt), gebracht.

**Fig.7** zeigt den Biosensor 700 für den Fall, dass in dem Elektrolyten DNA-Stränge 712 enthalten sind, die eine vorgegebene Nukleotidsequenz aufweisen, die komplementär ist zu der Sequenz der DNA-Sondenmoleküle 710.

Wie aus **Fig.7** ersichtlich ist, ist das Resultat, dass nach erfolgter Hybridisierung sich auf den Einheiten 701 hybridisierte Moleküle befinden, d.h. dort doppelsträngige DNA-Moleküle immobilisiert sind.

Mittels einer nicht gezeigten Lichtquelle (z.B. eines Lasers) wird daraufhin durch Pfeile 713 symbolisiertes Licht mit einer Wellenlänge eingestrahlt, die geeignet ist, die Markierungen 711 wie z.B. ein bakterielles Reaktionszentrum anzuregen. Es kommt dadurch zu einer photoinduzierten Ladungstrennung innerhalb der Cofaktoren des Reaktionszentrums und zu einer intermolekularen Elektronenübertragung.

Liegt an den (durch die Auswahlelektronik bestimmten) Einheiten zum Immobilisieren 701 ein geeignetes Potential an, kommt es zu einer Übertragung eines Elektrons von den doppelsträngigen Hybridmolekülen auf die Einheiten 701, d.h. zu einem Stromfluss, der durch die elektrische Erfassungsschaltung 704 erfasst wird.

Nun wird die Temperatur auf den ausgewählten Einheiten 701 durch die entsprechenden Thermostatisierungseinheiten 708 auf eine Temperatur oberhalb der Schmelztemperatur Tm erhöht. Dadurch kommt es zu einer Denaturierung und Trennung in die Einzelstränge, und somit auch zu einer Unterbrechung des Stromflusses, da die einzelsträngigen Moleküle 710 annähernd perfekte Isolatoren sind. Diese Änderung des Stromflusses wird durch die Schaltung 704 der Erfassungseinheit anhand einer zweiten, vorzugsweise kontinuierlichen Messung erfasst. Durch den Vergleich der beiden elektrischen Messung wird die Anwesenheit der DNA-Moleküle 712 ermittelt.

Die Verwendung des hier beschriebenen Biosensors 700 erlaubt nicht nur eine sehr empfindliche, sondern auch eine individuelle (und örtlich aufgelöste) Erfassung von einer oder mehreren Einheiten zum Immobilisieren und bietet darüber hinaus eine deutliche Vereinfachung der gesamten Messanordnung.

In diesem Dokument sind folgende Veröffentlichungen zitiert:

[1] R. Hintsche et al., Microbiosensors Using Electrodes Made in Si-Technology, Frontiers in Biosensorics, Fundamental Aspects, edited by F. W. Scheller et al., Dirk Hauser Verlag, Basel, S. 267 - 283, 1997

[2] R. Hintsche et al., Microelectrode arrays and application to biosensing devices, Biosensors & Bioelectronics, Vol. 9, S. 697 - 705, 1994

[3] M. Paeschke et al, Voltammetric Multichannel Measurements Using Silicon Fabricated Microelectrode Arrays, Electroanalysis, Vol. 7, Nr. 1, S. 1 - 8, 1996

[4] P. van Gerwen, Nanoscaled Interdigitated Electrode Arrays for Biochemical Sensors, IEEE, International Conference on Solid-State Sensors and Actuators, Chicago, S.907 - 910, 16. - 19. Juni 1997

[5] N.L. Thompson, B.C. Lagerholm, Total Internal Reflection Fluorescence: Applications in Cellular Biophysics, Current Opinion in Biotechnology, Vol. 8, S. 58 - 64, 1997.

[6] WO 00/42217 A1

[7] J.G. Wetmur, DNA Probes: Applications of the Principles of Nucleic Acid Hybridization, Critical Reviews in Biochemistry and Molecular Biology, 26(3/4):227-259 (1991)

[8] E. Braun et al., DNA-templated assembly and electrode attachment of a conducting silver wire, Nature, Vol. 391, S. 775 - 778, 1998

[9] Roche Molecular Biochemicals, 1999 Biochemicals Catalog, S.99.

[10] DE 199 40 810 A1

[11] WO 01/75151 A2

[12] DE 199 16 921 A1

[13] WO 94/05414 A1

[14] US 5,648,213

[15] US 5,972,692

[16] DE 199 26 457 A

[17] US 6,168,948 B

**Bezugszeichenliste**

| | |
|---|---|
| 100 | Biosensor |
| 101 | Elektroden |
| 102 | elektrischer Anschluss |
| 103 | Isolatorsschicht |
| 104 | Einheiten zum Immobilisieren |
| 105 | Thermostatisierungseinheit |
| 106 | DNA-Sondenmoleküle |
| 107 | DNA-Moleküle |
| 108 | DNA-Sondenmoleküle |
| 109 | Elektrolyt |

| | |
|---|---|
| 200 | Biosensor |
| 201 | Elektrode |
| 202 | Elektrode |
| 203 | Isolatorschicht |
| 204 | elektrischer Anschluss |
| 205 | elektrischer Anschluss |
| 206 | DNA-Sondenmoleküle |
| 207 | Analyt |
| 208 | DNA-Stränge |

| | |
|---|---|
| 300 | Biosensor |
| 301 | Substratmaterial |
| 302 | halbleitende Schicht |
| 303 | halbleitende Schicht |
| 304 | Kanalbbereich |
| 305 | Oxidschicht |
| 306 | metallisches Gate |
| 307 | Einheit für Temperaturführung |
| 308 | Fängermoleküle |
| 309 | Elektrode |
| 310 | Elektrode |

| | |
|---|---|
| 320 | Biosensor |
| 321 | Fotodiode |
| 322 | Fotodiode |

323 Isolatorschicht
324 elektrischer Anschluss
325 elektrischer Anschluss
326 Oxidschicht
327 Einheit zum Immobilisieren
328 Einheit zum Immobilisieren
329 Elektrode
330 Elektrischer Anschluss
331 Thermostatisierungseinheit
332 Fängermoleküle
333 Nukleinsäuremoleküle
334 Fluorophor
335 Pfeile

400 Biosensor
401 Erste Elektrode
402 Zweite Elektrode
403 Isolatorschicht
404 Haltebereich erste Elektrode
405 DNA-Sondenmolekül
406 zu untersuchende Lösung
407 DNA-Strang
408 Enzym
409 Spaltbares Molekül
410 Negativ geladenes erstes Teilmolekül
411 Pfeil
412 Weitere Lösung
413 Oxidiertes erstes Teilmolekül
414 Reduziertes erstes Teilmolekül

500 Diagramm
501 Y-Achse, elektrischer Strom
502 X-Achse, Zeit
503 Stromfluss
504 Anfangswert

600 Biosensor
601 leitfähige Sensoroberfläche
602 Fängermolekül
603 Markierung
604 Messgerät
605 zu erfassendes Nukleinsäuremolekül
606 Pfeil
607 Durch Pfeil symbolsierter Stromfluss

700 Biosensor
701 Einheiten zum Immobilisieren
702 Isolatorschicht
703 elektrische Anschlüsse
704 elektrische Erfassungsschaltung
705 Vorverstärker
706 Auswahlelektronik
707 Analog/Digitalwandler
708 Thermostatisierungseinheiten
709 elektrische Verbindungen
710 DNA-Sondenmoleküle
711 Redoxaktive Markierung
712 DNA-Stränge

713     Pfeil


**Patentansprüche**

1.  Verfahren zum Erfassen von Nukleinsäuremolekülen (107, 333), die mit mindestens einem Bindungspartner reversibel einen Komplex ausbilden, bei dem die Wechselwirkungen zwischen den Bindungspartnern in einem sprunghaft verlaufenden Vorgang aufgebrochen werden, mittels mindestens einer Einheit (104, 306) zum Immobilisieren von Nukleinsäuremolekülen, bei dem die mindestens eine Einheit (104, 306) zum Immobilisieren von Nukleinsäuremolekülen mit ersten Molekülen (106, 108, 332) versehen ist, die als Fängermoleküle dienen,

    • bei dem eine Probe (109) mit der mindestens einen Einheit (104, 306) zum Immobilisieren von Nukleinsäuremolekülen in Kontakt gebracht wird, wobei die Probe (109) die Nukleinsäuremoleküle (107, 333) enthalten kann, und wobei die Nukleinsäuremoleküle (107, 333) oder die ersten Moleküle (106, 108, 332) eine Markierung (334) aufweisen, mittels der ein detektierbares Signal (335) erzeugt werden kann,
    • bei dem in der Probe (109) enthaltene Nukleinsäuremoleküle (107, 333) an den Fängermolekülen (106, 332) gebunden werden, wodurch Komplexe aus Fängermolekülen (106, 332) und Nukleinsäuremolekülen (107, 333) gebildet werden,
    • bei dem mittels der Markierung (334) die Aussendung eines Signals (335) angeregt wird;
    • bei dem das mittels der Markierung (334) ausgesandte Signal (335) erfasst wird,
    • bei dem die Komplexe aus Fängermolekülen (106, 332) und Nukleinsäuremolekülen (107, 333) sprunghaft getrennt werden, wodurch eine Veränderung der Intensität des ausgesendeten Signals (335) bewirkt wird, und
    • bei dem die Nukleinsäuremoleküle (107, 333) mittels der Veränderung der Intensität des Signals (335) erfasst werden.

2.  Verfahren zum Erfassen von Nukleinsäuremolekülen (107, 333), die mit mindestens einem Bindungspartner reversibel einen Komplex ausbilden, bei dem die Wechselwirkungen zwischen den Bindungspartnern in einem sprunghaft verlaufenden Vorgang aufgebrochen werden, mittels einer Elektrodenanordnung (100), die mindestens eine Einheit (104, 306) zur Immobilisierung von Nukleinsäuremolekülen (107, 333) aufweist, bei dem die mindestens eine Einheit (104, 306) zum Immobilisieren von Nukleinsäuremolekülen mit ersten Molekülen (106, 108, 332) versehen ist, die als Fängermoleküle dienen,

    • bei dem eine Probe (109) mit der mindestens einen Einheit zum Immobilisieren in Kontakt gebracht wird, wobei die Probe die Nukleinsäuremoleküle (107, 333) enthalten kann,
    • bei dem in der Probe (109) enthaltene Nukleinsäuremoleküle (107, 333) an den Fängermolekülen (106, 332) gebunden werden, wodurch Komplexe aus Fängermolekülen (106, 332) und Nukleinsäuremolekülen (107, 333) gebildet werden,
    • bei dem eine erste elektrische Messung durchgeführt wird,
    • bei dem die Komplexe aus Fängermolekülen und Nukleinsäuremolekülen sprunghaft getrennt werden, wodurch eine Veränderung des Wertes der elektrischen Messung bewirkt wird,
    • bei dem nach der Trennung eine zweite elektrische Messung durchgeführt wird, und
    • bei dem die Nukleinsäuremoleküle (107, 333) mittels der Veränderung des Werts der elektrischen Messung erfasst werden.

3.  Verfahren nach Anspruch 2,
    bei dem bei der elektrischen Messung ein Potential an einer Elektrode (101), die Kapazität, der elektrische Widerstand, der elektrische Stromfluss oder das elektrische Potential an der Grenzfläche zwischen der Einheit zum Immobilisieren und dem Medium gemessen wird.

4.  Verfahren nach Anspruch 1 oder 2,
    bei dem statt Nukleinsäuremolekülen Komplexe aus Nukleinsäuren und Proteinen erfasst werden.

5.  Verfahren nach Anspruch 4,
    bei dem als Fängermoleküle Nukleinsäuremoleküle verwendet werden, so dass doppelsträngige Hybridmoleküle als Komplexe gebildet werden.

6.  Verfahren nach Anspruch 5,
    bei dem die als Fängermoleküle dienenden ersten Nukleinsäuremoleküle einzelsträngige Moleküle sind.

7. Verfahren nach Anspruch 6,
bei dem die Trennung der Hybridmoleküle aus Fängermolekülen und zu erfassenden Nukleinsäuremolekülen durch Erhöhung der Temperatur auf eine Temperatur oberhalb des Schmelzpunkts der doppelsträngigen Hybridmoleküle bewirkt wird.

8. Verfahren nach Anspruch 7, bei dem die Temperatur durch eine Thermostatisierungseinheit (708) gesteuert wird, die in einen Biosensor (700) integriert ist, der die mindestens eine Einheit (701) zum Immobilisieren aufweist.

9. Verfahren nach einem der Ansprüche 1 bis 8,
bei dem die mindestens eine Einheit zum Immobilisieren benachbart zu mindestens einer Arbeitselektrode angeordnet ist.

10. Verfahren nach Anspruch 9,
bei dem die Einheit zum Immobilisieren oberhalb der Arbeitselektrode angeordnet ist.

11. Verfahren nach einem der Ansprüche 1 bis 10,
bei dem die mindestens eine Einheit zum Immobilisieren direkt auf einer Arbeitselektrode oder einer Photodiode aufgebracht ist.

12. Verfahren nach einem der Ansprüche 1 bis 11,
bei dem die mindestens eine Einheit (306) zum Immobilisieren als Arbeitselektrode ausgestaltet ist.

13. Verfahren nach einem der Ansprüche 9 bis 12,
bei dem an eine Arbeitselektrode ein negatives elektrisches Potential zur Trennung der Hybridmoleküle angelegt wird.

14. Verfahren nach einem der Ansprüche 9 bis 13,
bei dem an eine Arbeitselektrode ein positives elektrisches Potential zur Bildung von doppelsträngigen Hybridmolekülen aus Fängermolekülen und zu erfassenden Nukleinsäuremoleküle angelegt wird.

15. Verfahren nach einem der vorhergehenden Ansprüche,
bei dem mehrere Einheiten (701) zum Immobilisieren von Nukleinsäuremolekülen in einer regelmäßigen Anordnung angeordnet sind.

16. Verfahren nach einem der Ansprüche 1 oder 4 bis 15,
bei dem die Markierung aus der Gruppe ausgewählt wird, die aus Fluoreszenz-Farbstoffen, Chemilumineszenz-Markierungen, Enzymen und Enzym-Liganden besteht.

17. Verfahren nach Anspruch 15,
bei dem die Markierung ein Enzym ist, das einen Reduktions-/oxidations-Recycling-Vorgang bewirkt.

18. Biosensor (700) zum Erfassen von Nukleinsäuremolekülen (712), die mit mindestens einem Bindungspartner reversibel einen Komplex ausbilden, bei dem die Wechselwirkungen zwischen den Bindungspartnern in einem sprunghaft verlaufenden Vorgang aufgebrochen werden, mit

• mindestens einer Einheit (701) zum Immobilisieren von Nukleinsäuremolekülen,
• einem Array von Erfassungseinheiten (704), die derart ausgestaltet sind, dass Nukleinsäuremoleküle (712), die an auf der Einheit zum Immobilisieren aufgebrachte Fängermoleküle (710) gebunden sind, mittels der Erfassungseinheiten (704) erfasst werden, und
• mit einer Mehrzahl von in den Sensor unter dem Array von Erfassungseinheiten integrierten Thermostatisierungseinheiten (708), die zum Trennen von Komplexen aus Fängermolekülen (710) und mittels der Mehrzahl von Erfassungseinheiten (704) erfassten Nukleinsäuremolekülen (712) mittels lokalen Erhöhens der Temperatur an der Oberfläche der mindestens einen Einheit (701) zum Immobilisieren der Nukleinsäuremoleküle auf eine Temperatur oberhalb des Schmelzpunkts der Komplexe eingerichtet sind, so dass eine individuelle und örtlich aufgelöste Erfassung der mindestens einen Einheit (701) zum Immobilisieren von Nukleinsäuremolekülen gewährleistet ist.

19. Biosensor nach Anspruch 18,

bei dem die mindestens eine Einheit (701) zum Immobilisieren oberhalb einer Arbeitselektrode angeordnet ist.

20. Biosensor nach Anspruch 18 oder 19,
bei dem die mindestens eine Einheit (701) zum Immobilisieren auf einer Arbeitselektrode oder einer Erfassungseinheit (704) aufgebracht ist.

21. Biosensor nach Anspruch 20,
bei dem die Erfassungseinheiten (704) Photodioden, CMOS-Kameras oder CCD-Kameras sind.

22. Biosensor nach Anspruch 18 bis 21,
bei der die mindestens eine Einheit (701) zum Immobilisieren auf einem CMOS-Chip aufgebracht ist.

23. Biosensor nach Anspruch 18,
bei dem die mindestens eine Einheit (701) zum Immobilisieren als Arbeitselektrode ausgestaltet ist.

24. Biosensor nach einem der Ansprüche 18 bis 23,
der mehrere Einheiten zum Immobilisieren von Nukleinsäuremolekülen in einer regelmäßigen Anordnung aufweist.

25. Biosensor (700) nach einem der Ansprüche 18 bis 24,
bei dem unter jeder Erfassungseinheit (704) eine Thermostatisierungseinheit (708) angeordnet ist, die jeweils individuell ansteuerbar und regelbar ist.

**Claims**

1. A method for detecting nucleic acid molecules (107, 333) reversibly forming a complex with at least one linkage partner, in which the interactions between the linkage partners are broken open in an discontinuously running process using at least one unit (104, 306) for immobilizing nucleic acid molecules, in which the at least one unit (104, 306) for immobilizing nucleic acid molecules is provided with first molecules (106, 108, 332) which serve as capture molecules,

   • in which a sample (109) is brought into contact with the at least one unit (104, 306) for immobilizing nucleic acid molecules, it being possible for the sample (109) to contain the nucleic acid molecules (107, 333), and the nucleic acid molecules (107, 333) or the first molecules (106, 108, 332) having a marking (334) which can be used to generate a detectable signal (335),
   • in which nucleic acid molecules (107, 333) contained in the sample (109) are bound to the capture molecules (106, 332), thereby forming complexes comprising capture molecules (106, 332) and nucleic acid molecules (107, 333),
   • in which the emission of a signal (335) is excited by means of the marking (334),
   • in which the signal (335) emitted by means of the marking (334) is detected,
   • in which the complexes comprising capture molecules (106, 332) and nucleic acid molecules (107, 333) are discontinuously separated, thereby altering the intensity of the emitted signal (335), and
   • in which the nucleic acid molecules (107, 333) are detected by means of the alteration of the intensity of the signal (335).

2. A method for detecting nucleic acid molecules (107, 333) reversibly forming a complex with at least one linkage partner, in which the interactions between the linkage partners are broken open in an discontinuously running process using an electrode arrangement (100) having at least one unit (104, 306) for immobilizing nucleic acid molecules (107, 333), in which the at least one unit (104, 306) for immobilizing nucleic acid molecules is provided with first molecules (106, 108, 332) which serve as capture molecules,

   • in which a sample (109) is brought into contact with the at least one immobilization unit, it being possible for the sample to contain the nucleic acid molecules (107, 333),
   • in which nucleic acid molecules (107, 333) contained in the sample (109) are bound to the capture molecules (106, 332), thereby forming complexes comprising capture molecules (106, 332) and nucleic acid molecules (107, 333),
   • in which a first electrical measurement is carried out,
   • in which the complexes comprising capture molecules and nucleic acid molecules are discontinuously separated, thereby altering the value of the electrical measurement,
- in which a second electrical measurement is carried out after the separation, and
- in which the nucleic acid molecules (107, 333) are detected by means of the alteration of the value of the electrical measurement.

3. The method as claimed in claim 2,
in which a potential at an electrode (101), the capacitance, the electrical resistance, the electrical current flow or the electrical potential at the interface between the immobilization unit and the medium is measured during the electrical measurement.

4. The method as claimed in claim 1 or 2,
in which instead of nucleic acid molecules, complexes comprising nucleic acids and proteins are detected.

5. The method as claimed in claim 4,
in which nucleic acid molecules are used as capture molecules so that double-stranded hybrid molecules are formed as complexes.

6. The method as claimed in claim 5,
in which the first nucleic acid molecules serving as capture molecules are single-stranded molecules.

7. The method as claimed in claim 6,
in which the separation of the hybrid molecules comprising capture molecules and nucleic acid molecules to be detected is effected by increasing the temperature to a temperature above the melting point of the double-stranded hybrid molecules.

8. The method as claimed in claim 7, in which the temperature is controlled by a thermostatically regulating unit (708) integrated into a biosensor (700) having the at least one immobilization unit (701).

9. The method as claimed in one of claims 1 to 8,
in which the at least one immobilization unit is arranged adjacent to at least one operating electrode.

10. The method as claimed in claim 9,
in which the immobilization unit is arranged above the operating electrode.

11. The method as claimed in one of claims 1 to 10,
in which the at least one immobilization unit is applied directly on an operating electrode or a photodiode.

12. The method as claimed in one of claims 1 to 11,
in which the at least one immobilization unit (306) is configured as an operating electrode.

13. The method as claimed in one of claims 9 to 12,
in which a negative electrical potential is applied to an operating electrode for the purpose of separating the hybrid molecules.

14. The method as claimed in one of claims 9 to 13,
in which a positive electrical potential is applied to an operating electrode for the purpose of forming double-stranded hybrid molecules comprising capture molecules and nucleic acid molecules to be detected.

15. The method as claimed in one of the preceding claims,
in which a plurality of units (701) for immobilizing nucleic acid molecules are arranged in a regular arrangement.

16. The method as claimed in one of claims 1 or 4 to 15,
in which the marking is selected from the group comprising fluorescent dyes, chemiluminescence markings, enzymes and enzyme ligands.

17. The method as claimed in claim 15,
in which the marking is an enzyme which effects a reduction/oxidation recycling process.

18. A biosensor (700) for detecting nucleic acid molecules (712) reversibly forming a complex with at least one linkage partner, in which the interactions between the linkage partners are broken open in an discontinuously running process, having

   • at least one unit (701) for immobilizing nucleic acid molecules,
   • an array of detection units (704) configured in such a way that nucleic acid molecules (712) bound to capture molecules (710) applied on the immobilization unit are detected by means of the detection units (704), and
   • having a plurality of thermostatically regulating units (708) which are integrated into the sensor below the array of detection units, which are set up for separating complexes comprising capture molecules (710) and nucleic acid molecules (712) detected by means of the plurality of detection units (704), by locally increasing the temperature at the surface of the at least one unit (701) for immobilizing nucleic acid molecules to a temperature above the melting point of the complexes such that an individual and locally resolved detection of the at least one unit (701) for immobilizing nucleic acid molecules is assured.

19. The biosensor as claimed in claim 18,
   in which the at least one immobilization unit (701) is arranged above an operating electrode.

20. The biosensor as claimed in claim 18 or 19,
   in which the at least one immobilization unit (701) is applied on an operating electrode or a detection unit (704).

21. The biosensor as claimed in claim 20,
   in which the detection units (704) are photodiodes, CMOS cameras or CCD cameras.

22. The biosensor as claimed in claims 18 to 21,
   in which the at least one immobilization unit (701) is applied on a CMOS chip.

23. The biosensor as claimed in claim 18,
   in which the at least one immobilization unit (701) is configured as an operating electrode.

24. The biosensor as claimed in one of claims 18 to 23,
   which has a plurality of units for immobilizing nucleic acid molecules in a regular arrangement.

25. The biosensor (700) as claimed in one of claims 18 to 24, in which a thermostatically regulating unit (708), which can be driven and regulated individually in each case, is arranged below each detection unit (704).

**Revendications**

1. Procédé de détection de molécules (107, 333) d'acide nucléique, qui forment un complexe avec au moins un partenaire de liaison, dans lequel on interrompt les interactions entre les partenaires de liaison dans un processus se déroulant brusquement à l'aide d'au moins une unité (104, 306) d'immobilisation de molécules d'acide nucléique, dans lequel on munit la au moins une unité (104, 306) d'immobilisation de molécules d'acide nucléique de premières molécules (106, 108, 332), qui servent de molécules de capture,

   • dans lequel on met un échantillon (109) en contact avec la au moins une unité (104, 306) d'immobilisation de molécules d'acide nucléique pouvant contenir les molécules (107, 333) d'acide nucléique et dans lequel les molécules (107, 333) d'acide nucléique ont un marquage (334) au moyen duquel un signal (335) détectable peut être produit,
   • dans lequel des molécules (107, 333) d'acide nucléique contenues dans l'échantillon (109) peuvent être liées aux molécules (106, 332) de capture en formant ainsi des complexes composés de molécules (106, 332) de capture et de molécules (107, 333) d'acide nucléique,
   • dans lequel on excite au moyen du marquage (334) l'émission d'un signal (335),
   • dans lequel on détecte le signal (335) émis au moyen du marquage (334),
   • dans lequel on sépare d'une manière brusque les complexes composés de molécules (106, 332) de capture et de molécules (107, 333) d'acide nucléique en provoquant ainsi une modification de l'intensité du signal (335) émis et
   • dans lequel on détecte les molécules (107, 333) d'acide nucléique au moyen de la modification de l'intensité du signal (335).

**2.** Procédé de détection de molécules (107, 333) d'acide nucléique, qui forment de manière réversible un complexe avec au moins un partenaire de liaison, dans lequel on interrompt dans un processus se déroulant brusquement les interactions entre les partenaires de liaison au moyen d'un dispositif (100) d'électrodes, qui a au moins une unité (104, 306) d'immobilisation de molécules (107, 333) d'acide nucléique, dans lequel la au moins une unité (104, 306) d'immobilisation de molécules d'acide nucléique est munie de premières molécules (106, 108, 332), qui servent de molécules de capture,

> • dans lequel on met un échantillon (109) en contact avec la au moins une unité d'immobilisation, l'échantillon pouvant contenir des molécules (107, 333) d'acide nucléique,
> • dans lequel des molécules (107, 333) d'acide nucléique contenues dans l'échantillon (109) sont liées aux molécules (106, 332) de capture en formant ainsi des complexes composés de molécules (106, 332) de capture et de molécules (107, 333) d'acide nucléique,
> • dans lequel on effectue une première mesure électrique,
> • dans lequel on sépare d'une manière brusque les complexes composés de molécules de capture et de molécules d'acide nucléique en provoquant ainsi une modification de la valeur de la mesure électrique,
> • dans lequel on effectue, après la séparation, une deuxième mesure électrique, et
> • dans lequel on détecte les molécules (107, 333) d'acide nucléique à l'aide de la modification de la valeur de la mesure électrique.

**3.** Procédé suivant la revendication 2,
dans lequel, lors de la mesure électrique, on mesure un potentiel sur une électrode (101), la capacité, la résistance électrique, le flux de courant électrique ou le potentiel électrique à l'interface entre l'unité d'immobilisation et le milieu.

**4.** Procédé suivant la revendication 1 ou 2,
dans lequel on détecte au lieu de molécules d'acide nucléique des complexes composés d'acides nucléiques et de protéines.

**5.** Procédé suivant la revendication 4,
dans lequel on utilise comme molécules de capture des molécules d'acide nucléique, de manière à former comme complexes des molécules hybrides à double brin.

**6.** Procédé suivant la revendication 5,
dans lequel les premières molécules d'acide nucléique servant de molécules de capture sont des molécules à un seul brin.

**7.** Procédé suivant la revendication 6,
dans lequel on provoque la séparation des molécules hybrides composées de molécules de capture et de molécules d'acide nucléique à détecter en élevant la température jusqu'à une température au-dessus du point de fusion des molécules hybrides à double brin.

**8.** Procédé suivant la revendication 7, dans lequel on règle la température par une unité (708) de thermostatisation, qui est intégrée dans un biocapteur (700), lequel a la au moins une unité (701) d'immobilisation.

**9.** Procédé suivant l'une des revendications 1 à 8,
dans lequel la au moins une unité d'immobilisation est disposée au voisinage d'une électrode de travail.

**10.** Procédé suivant la revendication 9,
dans lequel l'unité d'immobilisation est disposée au-dessus de l'électrode de travail.

**11.** Procédé suivant l'une des revendications 1 à 10,
dans lequel la au moins une unité d'immobilisation est disposée directement sur une électrode de travail ou sur une photodiode.

**12.** Procédé suivant l'une des revendications 1 à 11,
dans lequel la au moins une unité (306) d'immobilisation est conformée en électrode de travail.

**13.** Procédé suivant l'une des revendications 9 à 12,
dans lequel on applique, pour la séparation des molécules hybrides, un potentiel électrique négatif à l'électrode de travail.

**14.** Procédé suivant l'une des revendications 9 à 13,
dans lequel on applique un potentiel électrique positif à l'électrode de travail pour la formation de molécules hybrides à double brin composées de molécules de capture et de molécules d'acide nucléique à détecter.

**15.** Procédé suivant l'une des revendications précédentes,
dans lequel on dispose en un agencement régulier plusieurs unités (701) d'immobilisation de molécules d'acide nucléique.

**16.** Procédé suivant l'une des revendications 1 ou 4 à 15,
dans lequel on choisit le marquage dans le groupe qui est constitué de colorants de fluorescence, de marquage par chémioluminescence, d'enzymes et de ligands d'enzyme.

**17.** Procédé suivant la revendication 15,
dans lequel le marquage est un enzyme, qui provoque une opération de recyclage de réduction/oxydation.

**18.** Biocapteur (700) de détection de molécules (712) d'acide nucléique, qui forment de manière réversible un complexe avec au moins un partenaire de liaison, dans lequel les interactions entre les partenaires de liaison sont interrompues dans un processus se déroulant de manière brusque, comprenant

• au moins une unité (701) d'immobilisation de molécules d'acide nucléique,
• une matrice d'unités (704) de détection, qui sont conformées de manière à ce que des molécules (712) d'acide nucléique, qui sont liées à des molécules (710) de capture déposées sur l'unité d'immobilisation, soient détectées au moyen des unités (704) de détection, et
• comprenant une multiplicité d'unités (708) de thermostatisation, qui sont intégrées sur la matrice d'unités de détection et qui sont conçues pour la séparation de complexes composés de molécules (710) de capture et de molécules (712) d'acide nucléique détectées au moyen de la multiplicité d'unités (704) de détection au moyen d'une élévation locale de la température à la surface de la au moins une unité (701) d'immobilisation des molécules d'acide nucléique jusqu'à une température supérieure au point de fusion du complexe, de manière à assurer une détection individuelle et résolue dans l'espace de la au moins une unité (701) d'immobilisation de molécules d'acide nucléique.

**19.** Biocapteur suivant la revendication 18,
dans lequel la au moins une unité (701) d'immobilisation est disposée au-dessus d'une électrode de travail.

**20.** Biocapteur suivant la revendication 18 ou 19,
dans lequel la au moins une unité (701) d'immobilisation est déposée sur une électrode de travail ou sur une unité (704) de détection.

**21.** Biocapteur suivant la revendication 20,
dans lequel les unités (704) de détection sont des photodiodes, des caméras CMOS ou des caméras CDD.

**22.** Biocapteur suivant la revendication 18 à 21,
dans lequel la au moins une unité (701) d'immobilisation est déposée sur une puce CMOS.

**23.** Biocapteur suivant la revendication 18,
dans lequel la au moins une unité (701) d'immobilisation est conformée en électrode de travail.

**24.** Biocapteur suivant l'une des revendications 18 à 23,
qui a plusieurs unités d'immobilisation de molécules d'acide nucléique suivant un agencement régulier.

**25.** Biocapteur (700) suivant l'une des revendications 18 à 24,
dans lequel il est disposé sous chaque unité (704) de détection une unité (708) de thermostatisation, qui peut être commandée et régulée respectivement individuellement.

**FIG 1A**

**FIG 1B**

## FIG 2A

## FIG 2B

## FIG 3A

308

303    306
       305    302

       304

307    310    309    301
                            300
                     307

301

## FIG 3B

335
333    334
       327
323                335
                        328
                            326

331    321    329    322    331
                                320

324    323    330    325

## FIG 4A

## FIG 4B

## FIG 4C

## FIG 5

# FIG 6A

# FIG 6B

EP 1 444 357 B1

# FIG 6C

# FIG 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0042217 A1
- DE 19940810 A1
- WO 0175151 A2
- DE 19916921 A1
- WO 9405414 A1
- US 5648213 A
- US 5972692 A
- DE 19926457 A
- US 6168948 B

### In der Beschreibung aufgeführte Nicht-Patentliteratur

- **R. Hintsche et al.** Microbiosensors Using Electrodes Made in Si-Technology, Frontiers in Biosensorics, Fundamental Aspects. Dirk Hauser Verlag, 1997, 267-283
- **R. Hintsche et al.** Microelectrode arrays and application to biosensing devices. *Biosensors & Bioelectronics,* 1994, vol. 9, 697-705
- **M. Paeschke et al.** Voltammetric Multichannel Measurements Using Silicon Fabricated Microelectrode Arrays. *Electroanalysis,* 1996, vol. 7 (1), 1-8
- **P. van Gerwen.** Nanoscaled Interdigitated Electrode Arrays for Biochemical Sensors. *IEEE, International Conference on Solid-State Sensors and Actuators,* 16. Juni 1997, 907-910
- **N.L. Thompson ; B.C. Lagerholm.** Total Internal Reflection Fluorescence: Applications in Cellular Biophysics. *Current Opinion in Biotechnology,* 1997, vol. 8, 58-64
- **J.G. Wetmur.** DNA Probes: Applications of the Principles of Nucleic Acid Hybridization. *Critical Reviews in Biochemistry and Molecular Biology,* 1991, vol. 26 (3/4), 227-259
- **E. Braun et al.** DNA-templated assembly and electrode attachment of a conducting silver wire. *Nature,* 1998, vol. 391, 775-778
- Biochemicals Catalog. Roche Molecular Biochemicals, 1999, 99